# EUROPEAN PATENT SPECIFICATION

(11) **EP 1 254 107 B1**
(45) Date of publication and mention of the grant of the patent: **24.08.2005**
(21) Application number: 00987454.6
(22) Date of filing: 22.12.2000
(51) Int. Cl.: C07C 255/55, C09K 19/20, C09K 19/30

(54) **BENZOIC ACID ESTERS, AND LIQUID-CRYSTALLINE MEDIUM**
BENZOESÄUREESTER UND FLÜSSIGKRISTALLMEDIUM
ESTERS D'ACIDE BENZOIQUE, ET MILIEU DE CRISTAL LIQUIDE

(30) Priority: 07.02.2000 EP 00102142
(43) Date of publication of application: 06.11.2002
(73) Proprietor: Merck Patent GmbH, 64293 Darmstadt (DE)
(72) Inventor: GOULDING, Mark, Ringwood, Hampshire BH24 1SH (GB); ADLEM, Kevin, Poole, Dorset BH14 9BN (GB); TANNER, Richard, Ludlow, Shropshire SY8 2AH (GB); IONESCU, Doina, Southampton, Hampshire SO17 1ED (GB); FRANCIS, Matthew, Southampton, Hampshire SO17 2FN (GB); COATES, David, Wimborne, Dorset BH21 1SW (GB); REIFFENRATH, Volker, 64380 Rossdorf (DE); HIRSCHMANN, Harald, 64291 Darmstadt (DE)
(86) International application number: PCT/EP2000/013239
(87) International publication number: WO 2001/058858

(56) References cited:
- EP-A- 0 919 536
- DE-A- 3 906 021
- GB-A- 2 216 523
- KELLY, STEPHEN M.: "The synthesis and transition temperatures of benzoate ester derivatives o 2-fluoro-4-hydroxy- and 3-fluoro-4-hydroxybenzonitriles" HELV. CHIM. ACTA (1984), 67(6), 1572-9 , 1984, XP000990929
- KELLY, STEPHEN M. ET AL: "The synthesis and transition temperatures of ester derivatives of 2-fluoro-4-hydroxy- and 3-fluoro-4-hydroxybenzonitriles also incorporatin aliphatic ring systems" HELV. CHIM. ACTA (1984), 67(6), 1580-7 , 1984, XP000990930
- KELLY, STEPHEN M.: "Some novel nematic 4-cyanophenyl esters incorporating a lateral substituent" J. CHEM. SOC., CHEM. COMMUN. (1983), (7), 366-7 , 1983, XP000990928
- BEGUIN, A. ET AL: "Liquid crystals with perfluoroaromatic rings" J. PHYS., COLLOQ. (ORSAY, FR.) (1979), (3), 9-14 , 1979, XP000989470

## Description

The invention relates to novel benzoic acid esters of the formula I 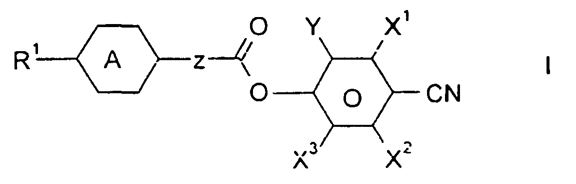 in which
- R¹: is H, an alkyl radical having 1-12 carbon atoms which is unsubstituted or at least monosubstituted by halogen or CN and in which, in addition, one or more CH₂ groups may each, independently of one another, be replaced by -O-, -C≡C- or -CH=CH- in such a way that heteroatoms are not connected directly, 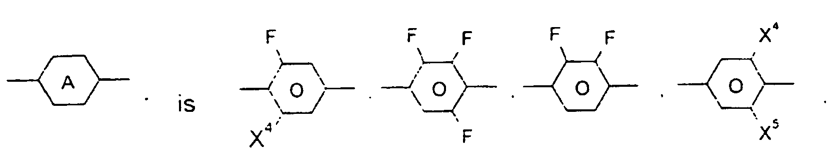 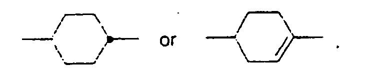
Z is -C≡C- or a single bond,
Y is H or F,
and
X¹, X¹, X³, X⁴ and X⁵, independently of one another, are H or F, with the proviso that, in the compounds of the formula I in which 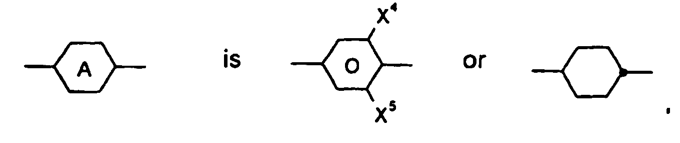 Y has the meaning F, and
a)
   - R¹: is an alkyl, oxaalkyl or alkoxy radical having 1 to 10 carbon atoms in which one or more CH₂ groups have been replaced by -C≡C-
   and/or
b)
   - Z: is -C≡C-.

The invention also relates to the use of the compounds of the formula I as components of liquid-crystalline media, and to liquid-crystal and electro-optical display elements which contain the liquid-crystalline media according to the invention.

The compounds of the formula I frequently have highly positive values of the dielectric anisotropy with moderate to high values of the optical anisotropy Δn and relatively low viscosity and can be used as components of liquid-crystalline media, in particular for displays based on the principle of the twisted cell, the guest-host effect, the effect of deformation of aligned phases (DAP) or electrically controlled birefringence (ECB) or the effect of dynamic scattering.

The substances employed hitherto for this purpose all have certain disadvantages, for example inadequate stability to the action of heat, light or electric fields, or unfavourable elastic and/or dielectric properties.

The invention had the object of finding novel, stable, liquid-crystalline or mesogenic compounds of particularly high positive dielectric anisotropy and moderate to high optical anisotropy at the same time as low viscosity which are suitable as components of liquid-crystalline media, in particular for TN, TFT and STN displays.

It has now been found that the compounds of the formula I are eminently suitable as components of liquid-crystalline media. They can be used to obtain stable liquid-crystalline media, in particular suitable for TFT or STN displays. The novel compounds are distinguished, in particular, by high thermal stability, which is advantageous for a high "holding ratio", and exhibit favourable clearing point values.

The provision of compounds of the formula I very generally considerably broadens the range of liquid-crystalline substances which are suitable, from various applicational points of view, for the preparation of liquid-crystalline mixtures.

The compounds of the formula I have a broad range of applications. Depending on the choice of substituents, these compounds can serve as base materials of which liquid-crystalline media are predominantly composed; however, it is also possible to add compounds of the formula I to liquid-crystalline base materials from other classes of compound in order, for example, to modify the dielectric and/or optical anisotropy of a dielectric of this type and/or to optimize its threshold voltage and/or its viscosity. Addition of compounds of the formula I to liquid-crystalline dielectrics allows the Δn values and Δε values of such media to be significantly affected.

The meaning of the formula I covers all isotopes of the chemical elements bound in the compounds of the formula I. In enantiomerically pure or enriched form, the compounds of the formula I are also suitable as chiral dopants and in general for producing chiral mesophases.

In the pure state, the compounds of the formula I are colourless and form liquid-crystalline mesophases in a temperature range which is favourably located for electro-optical use. They are stable chemically, thermally and to light.

The invention thus relates to the compounds of the formula I and to the use of these compounds as components of liquid-crystalline media. The invention furthermore relates to liquid-crystalline media comprising at least one compound of the formula I, and to liquid-crystal display elements, in particular electro-optical display elements, which contain media of this type.

Above and below, R¹, X¹, X², X³, X⁴, X⁵, Y and 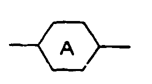 are as defined above unless expressly stated otherwise.

The following group of compounds of the sub-formulae IA to IX represents preferred embodiments of the invention:

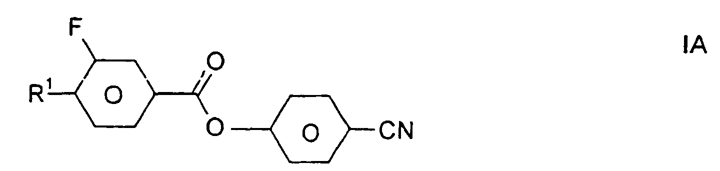

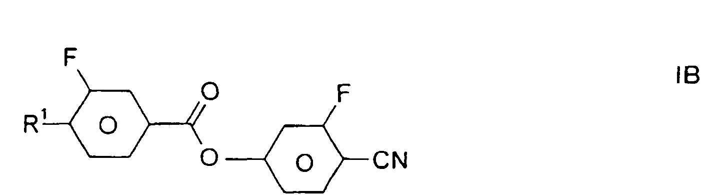

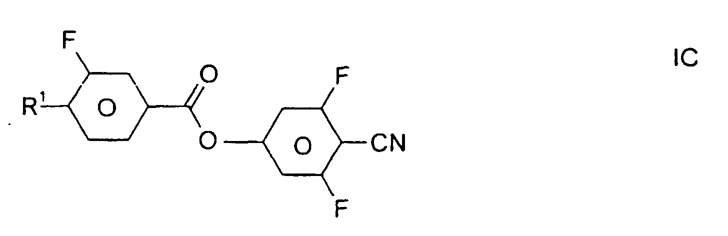

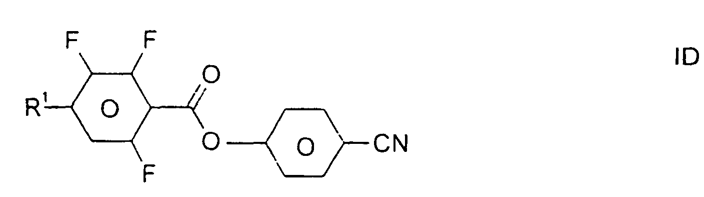

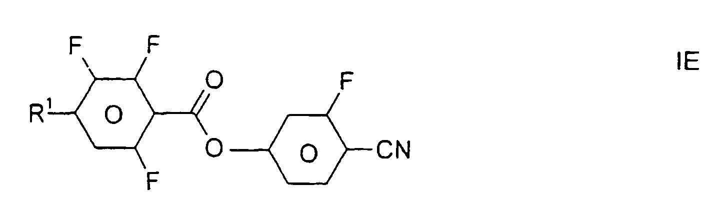

DE-A-3906021; GB-A-2216523; Kelly et al., Helv.Chim.Acta (1984), 67(6), 1572-79; Kelly et al., Helv.Chim.Acta (1984), 67(6),1580-87; Kelly et al., J.Chem.Soc.,Chem.Comm. (1983), (7), 366-67; and Beguin et al., J. Phys. Colloq. (1979), (3), 9-14 disclose fluorinated 4-cyano phenyl benzoates, but do not disclose compounds as claimed in the present invention. 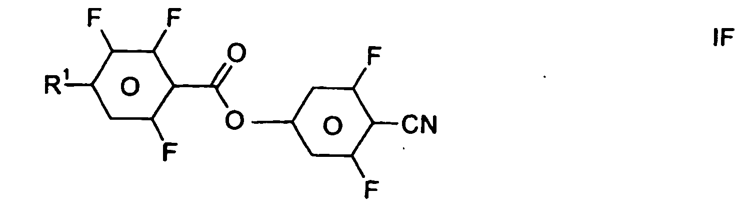 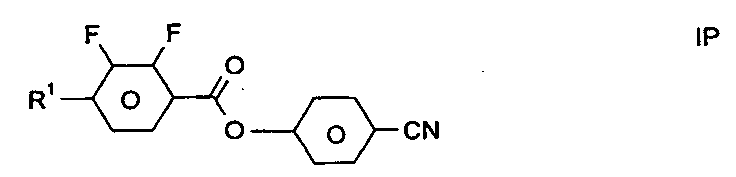 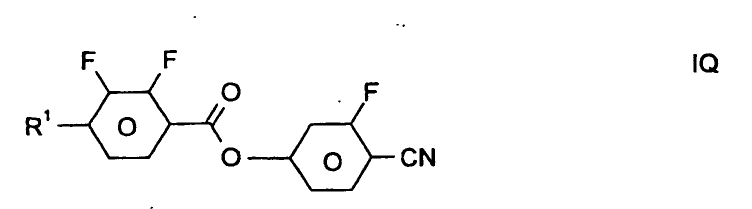 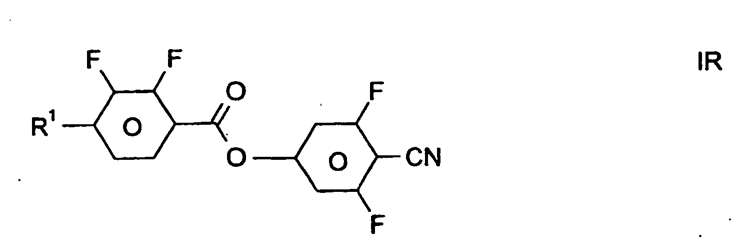 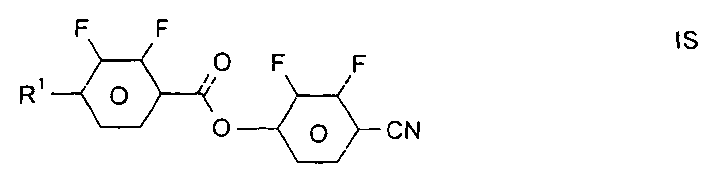 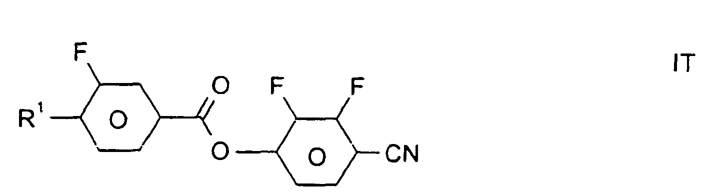 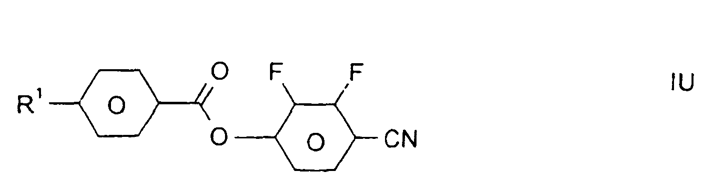 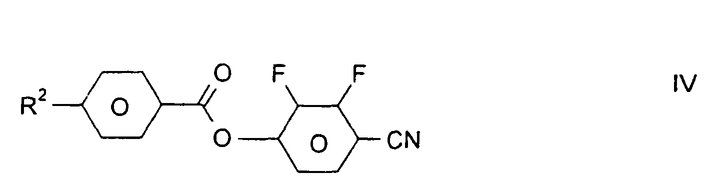 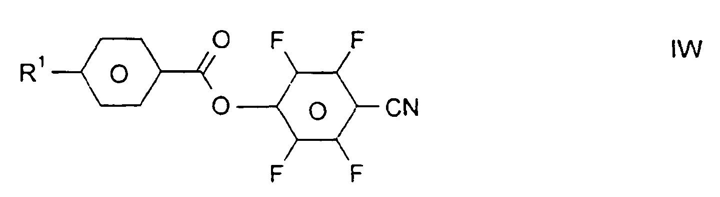 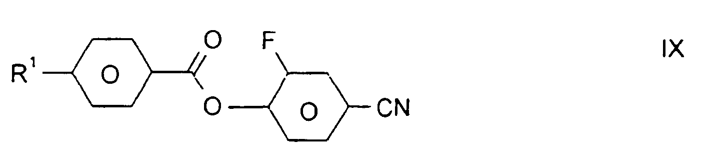 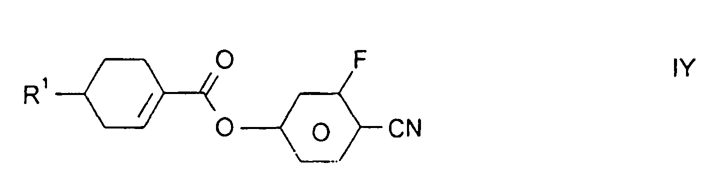 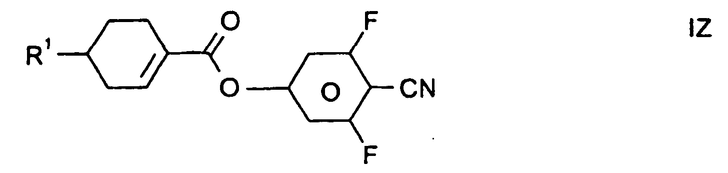 in which R¹ is as defined above, and R² is an alkyl, oxaalkyl or alkoxy radical having 1 to 10 carbon atoms in which one or more CH₂ groups have been replaced by -C≡C-.

If R¹ in the formulae above and below is an alkyl radical, this can be straight-chain or branched.

It is preferably straight-chain, has 2, 3, 4, 5, 6, or 7 carbon atoms and accordingly is preferably ethyl, propyl, butyl, pentyl, hexyl or heptyl, furthermore methyl, octyl, nonyl, decyl, undecyl, dodecyl, tridecyl, tetradecyl or pentadecyl.

If R¹ is an alkyl radical in which one CH₂ group has been replaced by -O-, this can be straight-chain or branched. It is preferably straight-chain and has 1 to 10 carbon atoms. Preferably, the first CH₂ group of this alkyl radical has been replaced by -O-, so that the radical R¹ becomes alkoxy and is preferably methoxy, ethoxy, propoxy, butoxy, pentyloxy, hexyloxy, heptyloxy, octyloxy or nonyloxy.

Furthermore, one CH₂ group elsewhere can also be replaced by -O-, so that the radical R¹ is preferably straight-chain 2-oxapropyl (= methoxymethyl), 2- (= ethoxymethyl) or 3-oxabutyl (= 2-methoxyethyl); 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanonyl, or 2-, 3-, 4-, 5-, 6-, 7-, 8- or 9-oxadecyl.

If R¹ is an alkenyl radical, this can be straight-chain or branched. It is preferably straight-chain and has 2 to 10 carbon atoms. Accordingly, it is in particular vinyl, prop-1- or -2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5-, -6- or -7-enyl, non-1-, -2-, -3-, -4-, -5-, -6-, -7- or -8-enyl, or dec-1-, -2-, -3-, -4-, -5-, -6-, -7-, -8- or -9-enyl.

R¹ is particularly preferably an alkenyl radical from the following group:

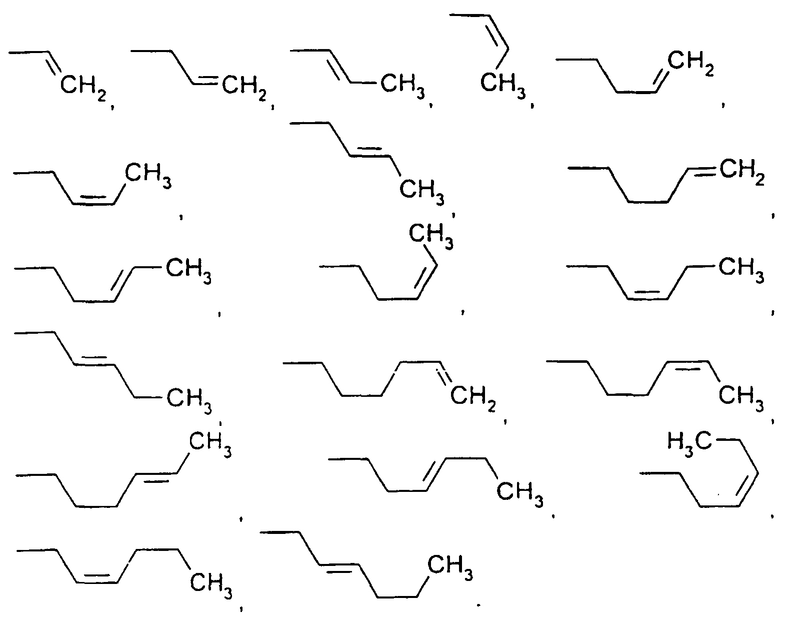

If R¹ is an alkenyloxy radical, this can be straight-chain or branched. It is preferably straight-chain and has 2 to 10 carbon atoms. It is particularly preferably a radical from the following group:

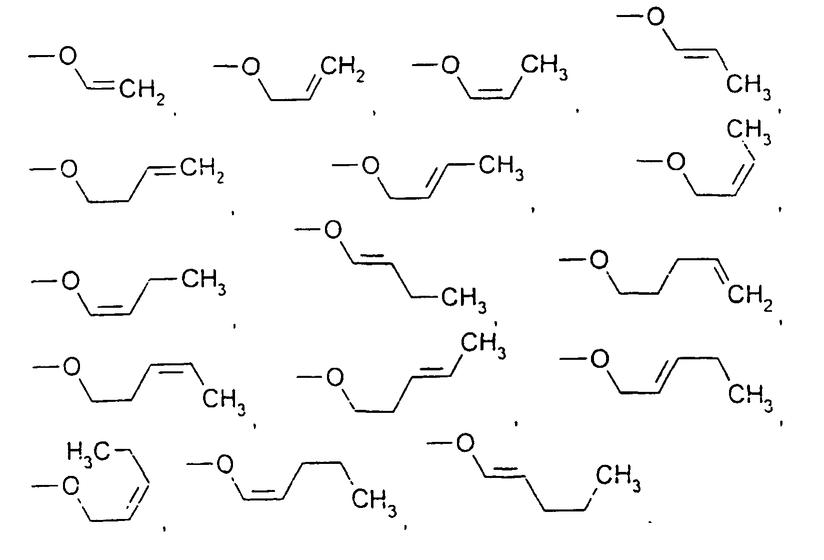

If R¹ is an alkyl radical which is at least monosubstituted by halogen, this radical is preferably straight-chain. Halogen is preferably F or Cl. In the case of polysubstitution, halogen is preferably F. The resultant radicals also include perfluorinated radicals. In the case of monosubstitution, the fluorine or chlorine substituent can be in any desired position, but is preferably in the ω-position.

If R¹ or R² is an alkynyl radical, this can be straight-chain or branched. It is preferably straight-chain and has 2 to 10 carbon atoms. Accordingly, it is in particular ethynyl, prop-1- or -2-ynyl, but-1-, -2- or -3-ynyl, pent-1-, -2-, -3-or -4-ynyl, hex-1-, -2-, -3-, -4- or -5-ynyl, hept-1-, -2-, -3-, -4-, -5- or -6-ynyl, oct-1-, -2-, -3-, -4-, -5-, -6- or -7-ynyl, non-1-, -2-, -3-, -4-, -5-, -6-, -7- or -8-ynyl, or dec-1-. -2-, -3-, -4-. -5-, -6-, -7-, -8- or -9-ynyl.

R¹ is particularly preferably a radical from the following group:

-C≡CH, -CH₂-C≡CH, -C≡C-CH₃, -C≡C-C₂H₅, -CH₂-CH₂-CH≡CH, -CH₂-C≡C-CH₃, -CH₂-CH₂-C≡C-CH₃ and -CH₂-CH₂-C≡C-C₂H₅.

If R¹ or R² is an oxaalkyl or alkoxy radical in which a CH₂ group has been replaced by -C≡C-, this can be straight-chain or branched. It is preferably straight-chain and has 2 to 10 carbon atoms. It is in particular a radical from the following group:

-O-CH₂-C≡CH, -O-CH₂-C≡C-CH₃, -O-CH₂-CH₂-C≡CH, -O-CH₂-CH₂-C≡C-CH₃, -O-CH₂-CH₂-C≡C-C₂H₅, -C≡C-CH₂-O-CH₃, -C≡C-CH₂-OC₂H₅ or -C≡C-CH₂CH₂-O-CH₃.

Compounds of the formula I containing a branched wing group R¹ may occasionally be of importance owing to better solubility in the customary liquid-crystalline base materials, but in particular as chiral dopants if they are optically active. Smectic compounds of this type are suitable as components of ferroelectric materials.

Branched groups of this type generally contain not more than one chain branch. Preferred branched radicals R¹ are isopropyl, 2-butyl (= 1-methylpropyl), isobutyl (= 2-methylpropyl), 2-methylbutyl, isopentyl (= 3-methylbutyl), 2-methylpentyl, 3-methylpentyl, 2-ethylhexyl, 2-propylpentyl, isopropoxy, 2-methylpropoxy, 2-methylbutoxy, 3-methylbutoxy, 2-methylpentoxy, 3-methylpentoxy, 2-ethylhexyloxy, 1 methylhexyloxy or 1-methylheptyloxy.

Formula I covers the racemates of these antipodes, and mixtures thereof.

Of these compounds of the formula I and th given to those in which at least one of the radicals presen therein has one of the preferred meanings indicated.

Further preferred compounds of the formula I

Compounds of the formula I whose R¹ group radical having 1 to 10 carbon atoms in which been replaced by -C≡C-.

Compounds of the formula I in which one of two radicals X¹ and X² is H and the other is F or in which the radicals are simultaneously F.

Compounds of the formula I in which both radicals X¹ and X⁴ are simultaneously F.

Compounds of the formula I in which Y is F.

Compounds of the formula I in which the ring 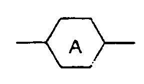 is

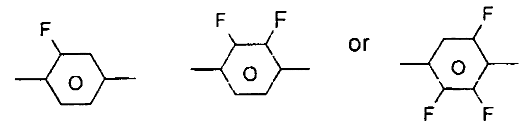

Compounds of the formula I in which Z is a single bond.

Some very particularly preferred smaller groups of compounds of the formula I are those of the sub-formulae I1 to I21: 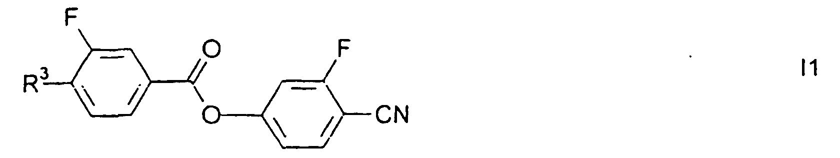 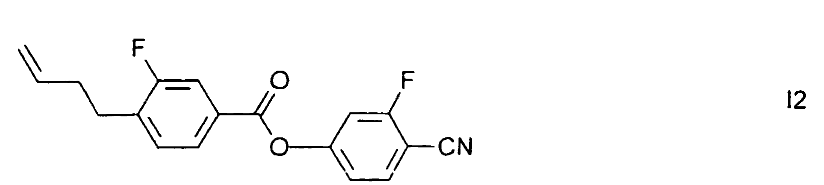 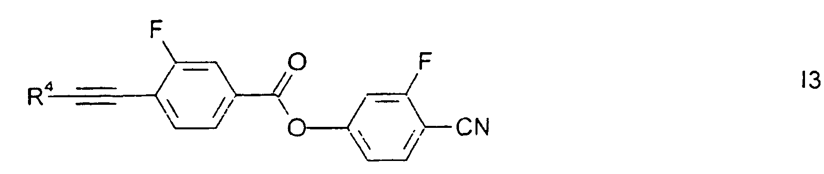 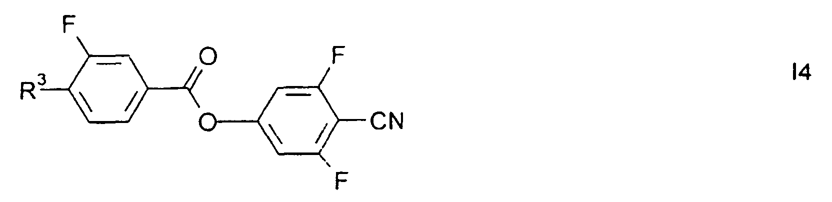 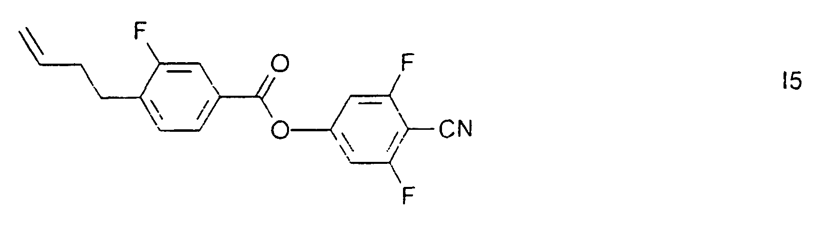 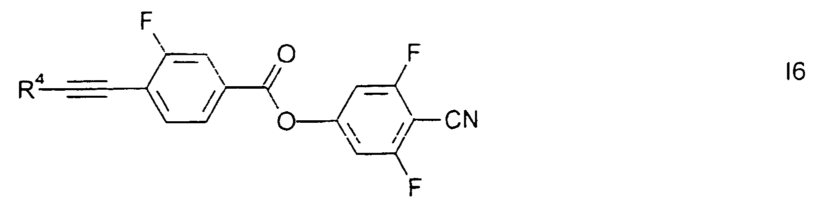 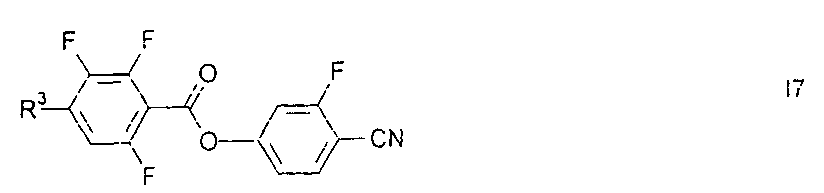 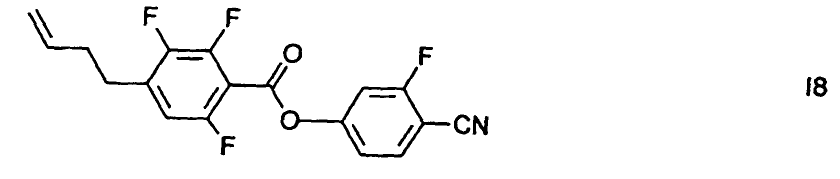 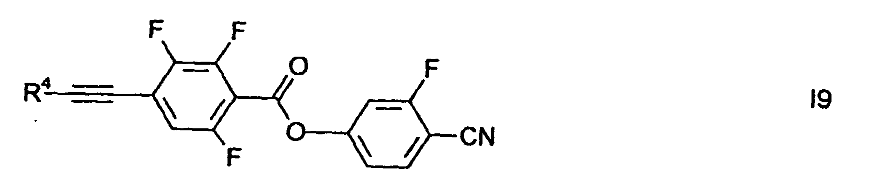 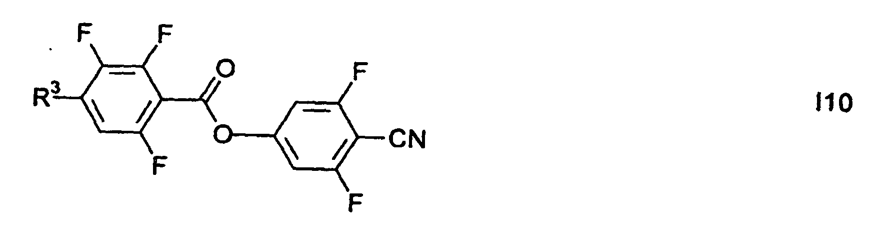 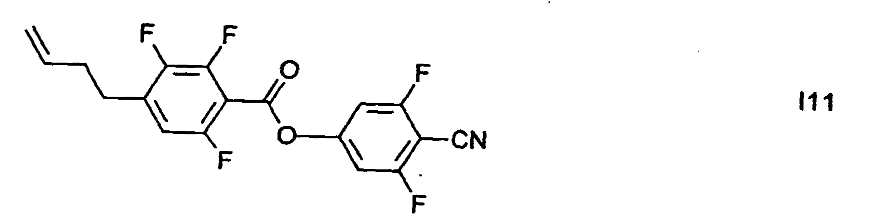 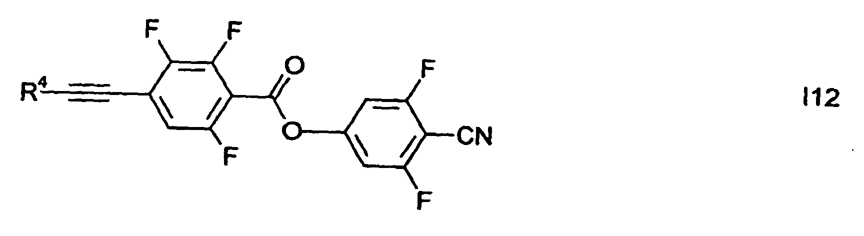 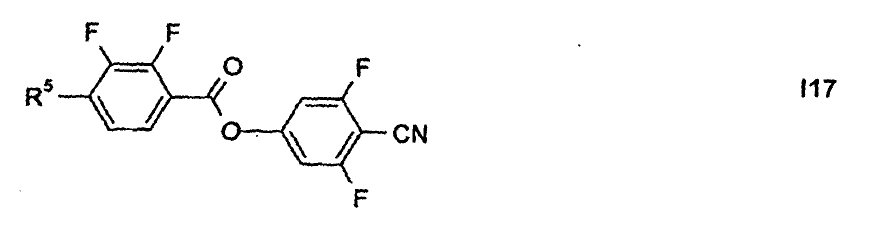 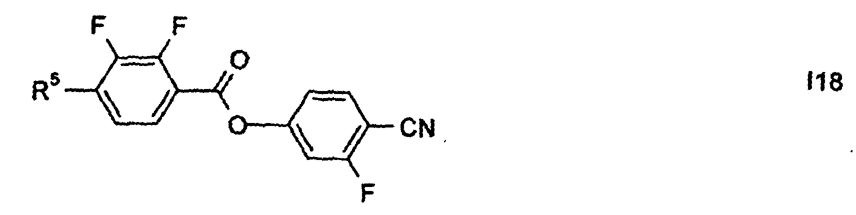 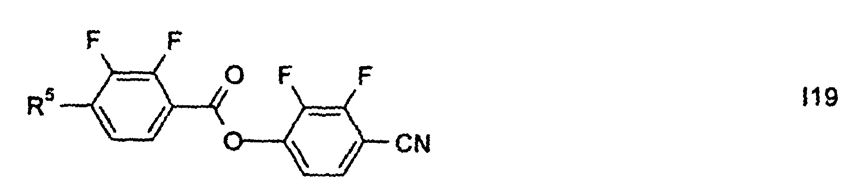 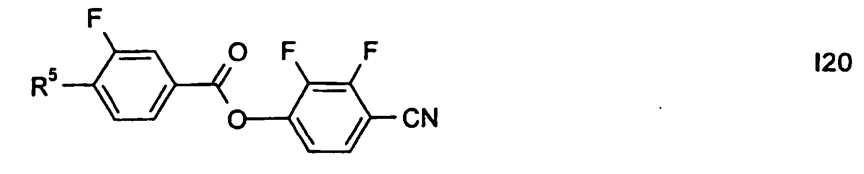 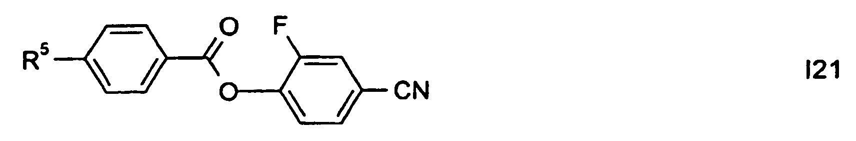 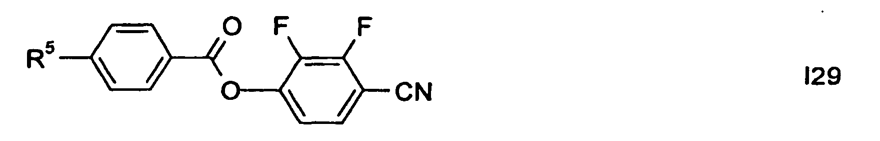 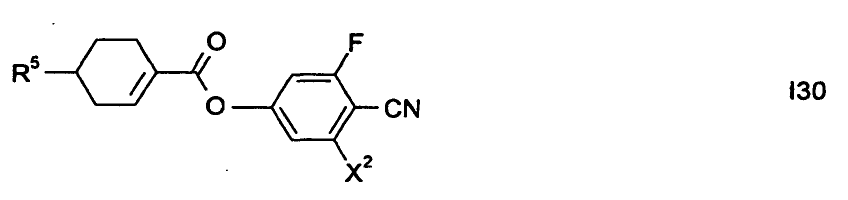 in which X² and X⁴ are as defined above. R³ is alkyl having 1 to 10 carbon atoms, preferably n-alkyl having 1 to 7 carbon atoms. R⁴ is H or alkyl or oxaalkyl having 1 to 7 carbon atoms, preferably n-alkyl having 1 to 5, very preferably 1 to 3 carbon atoms. R⁵ is alkyl or alkenyl having 2 to 7 carbon atoms, n is an integer from 1 to 7, preferably 1, 2, 3, 4 or 5.

Very particularly preferred compounds from this group are those of the formulae 12, I3, I6, I8, I12, I13 and I14. Further preferred are compounds of the formulae 122, 123, 124, I25, I26, I27 and I28.

The compounds of the formula I are prepared by methods known per se, as described in the literature (for example in the standard works, such as Houben-Weyl, Methoden der Organischen Chemie [Methods of Organic Chemistry], Georg-Thieme-Verlag, Stuttgart), to be precise, under reaction conditions which are known and suitable for said reactions.

Use can be made here of variants which are known per se, but are not mentioned here in greater detail.

The starting materials can, if desired, also be formed in situ by not isolating them from the reaction mixture, but instead immediately converting them further into the compounds of the formula I.

The compounds according to the invention can be prepared, for example, as shown in the following reaction schemes:

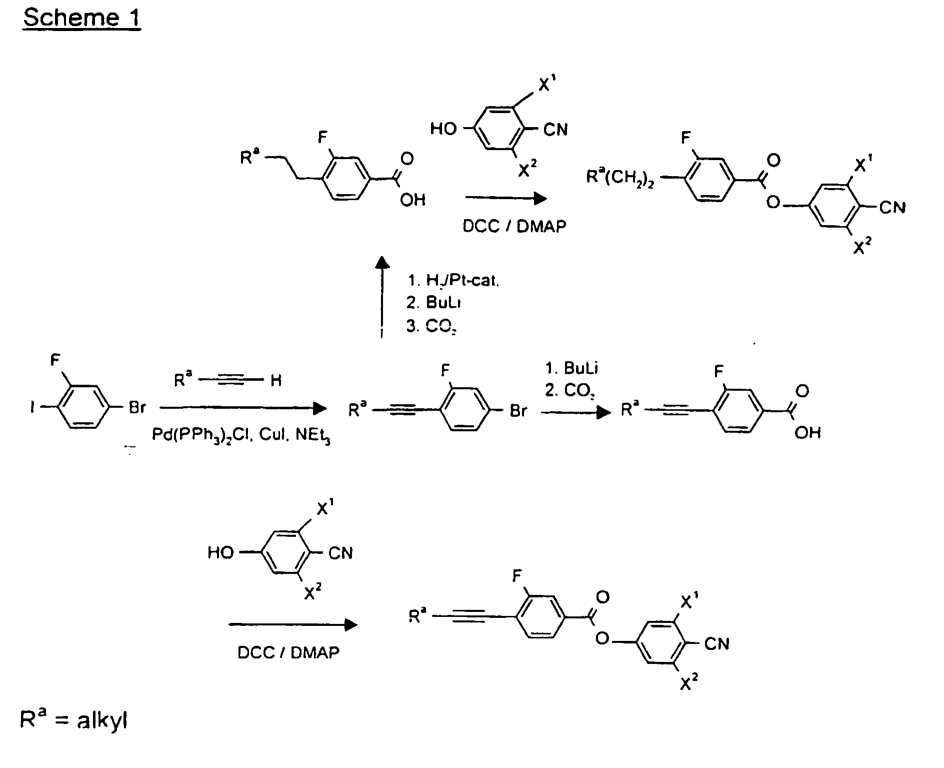

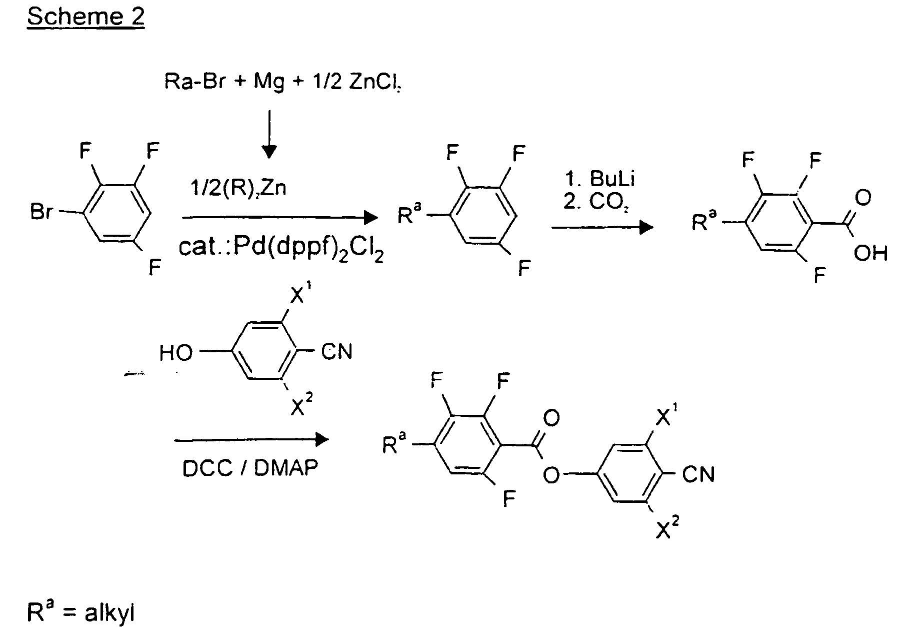

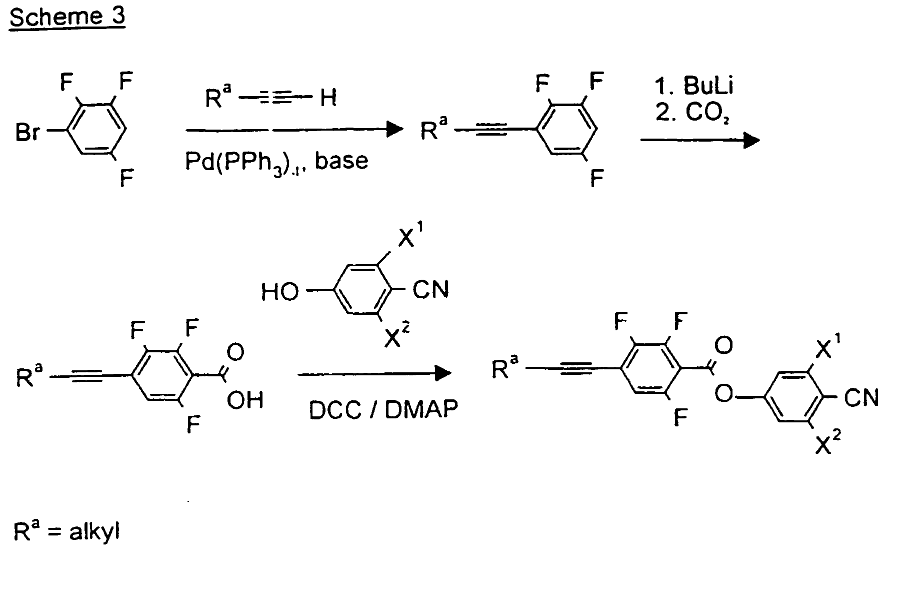

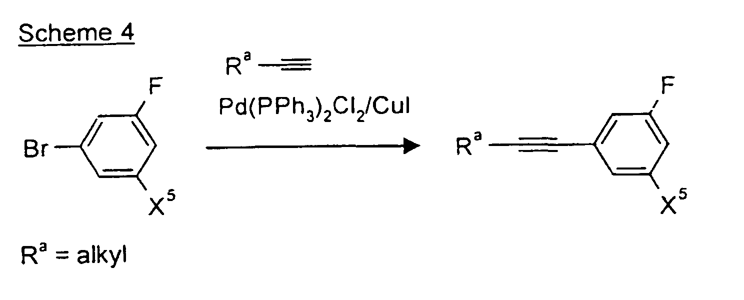

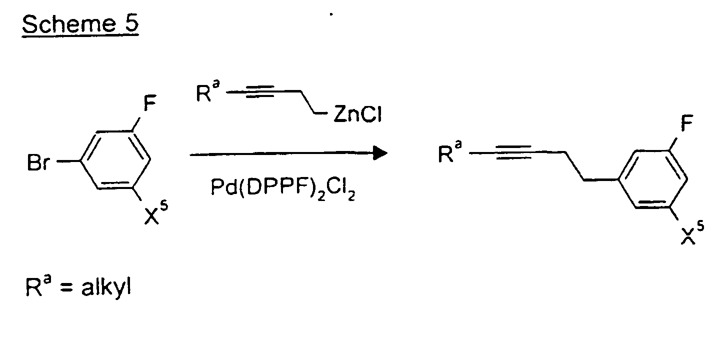

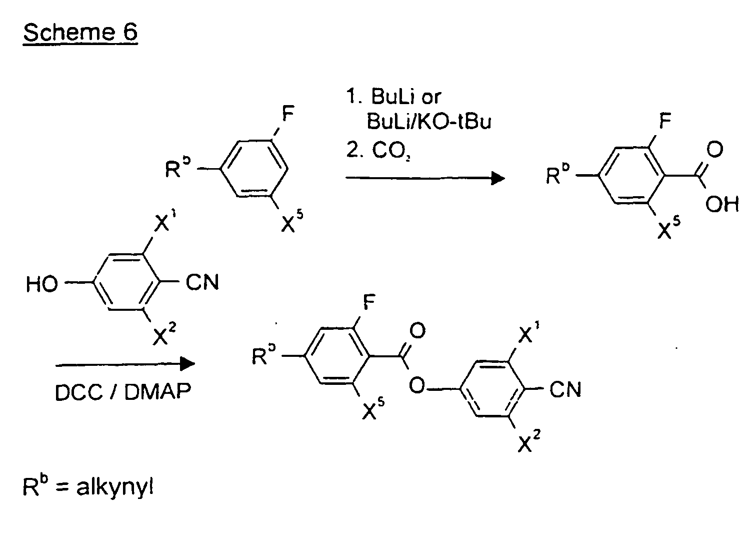

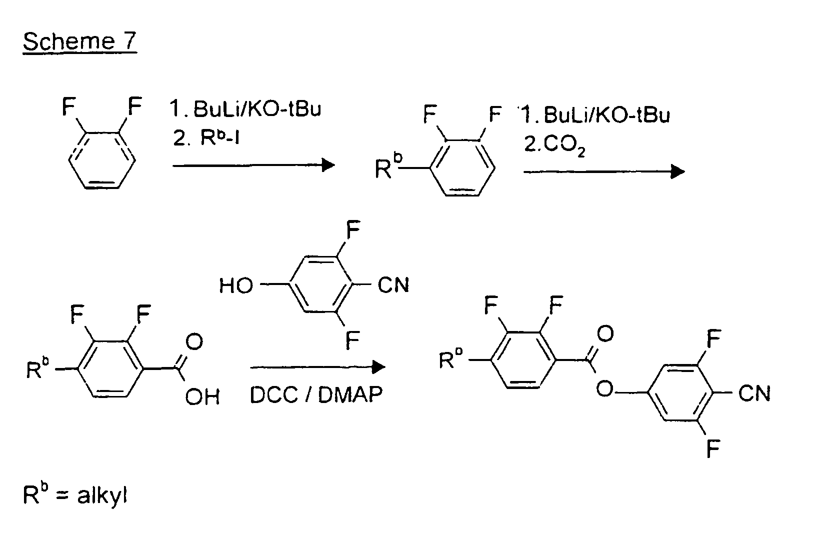

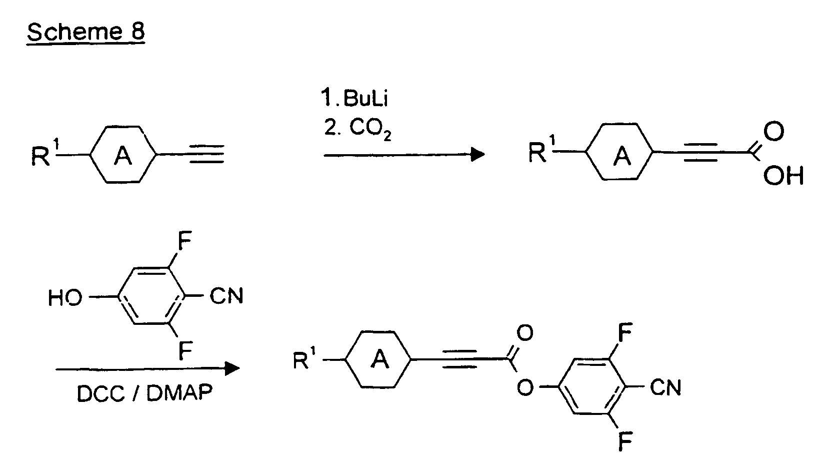

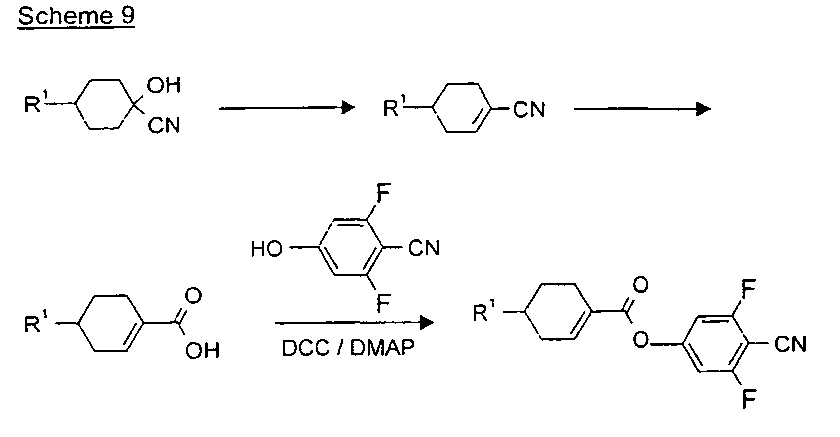

Esters of the formula I can also be obtained by esterification of corresponding carboxylic acids (or reactive derivatives thereof) using alcohols or phenols (or reactive derivatives thereof) or by the DCC method (DCC = dicyclohexylcarbodiimide).

The corresponding carboxylic acids and alcohols or phenols are known or can be prepared analogously to known processes.

Suitable reactive derivatives of said carboxylic acids are in particular the acid halides, especially the chlorides and bromides, furthermore the anhydrides, azides or esters, in particular alkyl esters having 1-4 carbon atoms in the alkyl group.

Suitable reactive derivatives of said alcohols and phenols are in particular the corresponding metal alkoxides and phenoxides respectively, preferably of an alkali metal, such as Na or K.

The esterification is advantageously carried out in the presence of an inert solvent. Particularly suitable solvents are ethers, such as diethyl ether, di-n-butyl ether, THF, dioxane or anisole, ketones, such as acetone, butanone or cyclohexanone, amides, such as DMF or hexamethylphosphoric triamide. hydrocarbons, such as benzene, toluene or xylene, halogenated hydrocarbons, such as tetrachloromethane or tetrachloroethytene, and sulfoxides, such as dimethyl sulfoxide or sulfolane. Water-immiscible solvents can at the same time advantageously be used for removal by azeotropic distillation of the water formed during the esterification. It may occasionally also be possible to use an excess of an organic base, for example pyridine, quinoline or triethylamine, as solvent for the esterification. The esterification can also be carried out in the absence of a solvent, for example by simply heating the components in the presence of sodium acetate. The reaction temperature is usually between -50° and +250°, preferably between -20° and +80°. At these temperatures, the esterification reactions are generally complete after from 15 minutes to 48 hours.

In detail, the reaction conditions for the esterification depend substantially on the nature of the starting materials used. Thus, the reaction of a free carboxylic acid with a free alcohol or phenol is generally carried out in the presence of a strong acid, for example a mineral acid, such as hydrochloric acid or sulfuric acid. A preferred reaction procedure is to react an acid anhydride or, in particular, an acid chloride with an alcohol, preferably in a basic medium, important bases being, in particular, alkali metal hydroxides such as sodium hydroxide or potassium hydroxide, alkali metal carbonates or hydrogencarbonates, such as sodium carbonate, sodium hydrogencarbonate, potassium carbonate or potassium hydrogencarbonate, alkali metal acetates, such as sodium acetate or potassium acetate, alkaline-earth metal hydroxides, such as calcium hydroxide, or organic bases, such as triethylamine, pyridine, lutidine, collidine or quinoline. A further preferred embodiment of the esterification comprises first converting the aicohoi or phenol into the sodium or potassium alkoxide or phenoxide, for example by treatment with ethanolic sodium hydroxide or potassium hydroxide solution, and isolating the product and reacting it with an acid anhydride or, in particular, acid chloride.

Nitriles can be obtained by replacement of halogens using copper cyanide or alkali metal cyanide.

In a further process for the preparation of compounds of the formula I in which R¹ is alkenyl, an aryl halide is reacted with an olefin in the presence of a tertiary amine and in the presence of a palladium catalyst (R.F. Heck, Acc. Chem. Res 12 (1979) 146). Examples of suitable aryl halides are chlorides, bromides and iodides, in particular bromides and iodides. The tertiary amines necessary for the success of the coupling reaction, such as, for example, triethylamine, are also suitable as solvent. Examples of suitable palladium catalysts are its salts, in particular Pd(II) acetate, with organophosphorus(III) compounds, such as, for example, triarylphosphines. This process can be carried out in the presence or absence of an inert solvent at temperatures between about 0°C and 150°C, preferably between 20°C and 100°C; suitable solvents are, for example, nitriles, such as acetonitrile, or hydrocarbons, such as benzene or toluene. The aryl halides and olefins employed as starting materials are frequently commercially available and can be prepared by processes known from the literature, tor example by halogenation of corresponding parent compounds or by elimination reactions on corresponding alcohols or halides.

Ethers of the formula I are obtainable by etherification of corresponding hydroxyl compounds, preferably corresponding phenols, the hydroxyl compound advantageously first being converted into a corresponding metal derivative, for example into the corresponding alkali metal alkoxide or alkali metal phenoxide, by treatment with NaH, NaNH₂, NaOH, KOH, Na₂CO₃ or K₂CO₃. This metal derivative can then be reacted with the appropriate alkyl halide, alkyl sulfonate or dialkyl sulfate, advantageously in an inert solvent, such as for example, acetone, 1,2-dimethoxyethane, DMF or dimethyl sulfoxide, or alternatively with an excess of aqueous or aqueous-alcoholic NaOH or KOH, at temperatures between about 20°C and 100°C.

In order to prepare the laterally substituted fluorine or chlorine compounds of the formula I, corresponding aniline derivatives can be reacted with sodium nitrite and either with tetrafluoroboric acid (in order to introduce an F atom) or with copper(I) chloride (in order to introduce a chlorine atom), to give the diazonium salts, which are then decomposed thermally at temperatures of 100-140°

The organometallic compounds are prepared, for example, by metal-halogen exchange (for example in accordance with Org. React. 6, 339-366 (1951)) between the corresponding halogen compound and an organolithium compound, such as, preferably, tert-butyllithium or lithium naphthalenide, or by reaction with magnesium turnings.

The linking of an aliphatic group R¹ to an aromatic ring is preferably carried out by Friedel-Crafts alkylation or acylation by reacting the corresponding aromatic compounds with Lewis acid catalysis. Examples of suitable Lewis acids are SnCl₄, ZnCl₂ and in particular AlCl₃ and TiCl₄.

In addition, the compounds of the formula I can be prepared by reducing a compound which contains one or more reducible groups and/or C-C bonds in place of H atoms, but otherwise conforms to the formula I.

Suitable reducible groups are preferably carbonyl groups, in particular keto groups, furthermore, for example, free or esterified hydroxyl groups or aromatically bonded halogen atoms. Preferred starting materials for the reduction are compounds which conform to the formula I, but contain a - CH=CH- group in place of a -CH₂CH₂- group and/or contain a -CO- group in place of a -CH₂- group and/or contain a free or functionally derived (for example in the form of its p-toluenesulfonate) OH group in place of an H atom.

The reduction can be carried out, for example, by catalytic hydrogenation at temperatures between about 0° and about 200° and at pressures between about 1 and 200 bar in an inert solvent, for example an alcohol, such as methanol, ethanol or isopropanol, an ether, such as tetrahydrofuran (THF) or dioxane, an ester such as ethyl acetate, a carboxylic acid. such as acetic acid, or a hydrocarbon, such as cyclohexane. Suitable catalysts are advantageously noble metals, such as Pt or Pd, which may be employed in the form of oxides (for example PtO₂ or PdO), on a support (for example Pd on carbon, calcium carbonate or strontium carbonate) or in finely divided form.

Ketones can also be reduced by the methods of Clemmensen (using zinc, zinc amalgam or tin and hydrochloric acid, advantageously in aqueous-alcoholic solution or in the heterogeneous phase with water/toluene at temperatures between about 80 and 120°C) or Wolff-Kishner (using hydrazine, advantageously in the presence of alkali, such as KOH or NaOH, in a high-boiling solvent, such as diethylene glycol or triethylene glycol, at temperatures between about 100 and 200°C) to give the corresponding compounds of the formula I which contain alkyl groups and/or -CH₂CH₂-bridges.

Furthermore, reductions using complex hydrides are possible. For example, arylsulfonyloxy groups can be removed reductively using LiAlH₄, in particular p-toluenesulfonyloxymethyl groups can be reduced to methyl groups, advantageously in an inert solvent, such as diethyl ether or THF, at temperatures between about 0 and 100°C. Double bonds can be hydrogenated using NaBH₄ or tributyltin hydride in methanol.

The starting materials are either known or can be prepared analogously to known compounds.

The liquid-crystalline media according to the invention preferably comprise from 2 to 40 components, in particular from 4 to 30 components, as further constituents besides one or more compounds according to the invention. These media very particularly preferably comprise from 7 to 25 components besides one or more compounds according to the invention. These further constituents are preferably selected from nematic or nematogenic (monotropic or isotropic) substances, in particular substances from the classes of the azoxybenzenes, benzylideneanilines, biphenyls, terphenyls, phenyl or cyclohexyl benzoates, phenyl or cyclohexyl esters of cyclohexanecarboxylic acid, phenyl or cyclohexyl esters of cyclohexylbenzoic acid, phenyl or cyclohexyl esters of cyclohexylcyclohexanecarboxylic acid, cyclohexylphenyl esters of benzoic acid, of cyclohexanecarboxylic acid or of cyclohexylcyclohexanecarboxylic acid, phenylcyclohexanes, cyclohexylbiphenyls, phenylcyciohexylcyclohexanes, cyclohexylcyclohexanes, cyclohexylcyclohexylcyclohexenes, 1,4-bis-cyclohexylbenzenes, 4,4'-bis-cyclohexylbiphenyls, phenyl- or cyclohexyipyrimidines, phenyl- or cyciohexyipyridines, phenyl- or cyclohexyldioxanes, phenyl- or cyclohexyl- 1,3-dithianes, 1,2-diphenylethanes, 1,2-dicyclohexylethanes, 1-phenyl-2-cyclohexylethanes, 1-cyclohexyl-2-(4-phenylcyclohexyl)ethanes, 1-cyclohexyl-2-biphenylylethanes, 1-phenyl-2-cyclohexylphenylethanes optionally halogenated stilbenes, benzyl phenyl ethers, tolans and substituted cinnamic acids. The 1,4-phenylene groups in these compounds may also be fluorinated.

The most important compounds suitable as further constituents of media according to the invention can be characterized by the formulae 1, 2, 3, 4 and 5:

R'-L-E-R" 1

R'-L-COO-E-R" 2

R'-L-OOC-E-R" 3

R'-L-CH₂CH₂-E-R" 4

R'-L-C≡C-E-R" 5

In the formulae 1, 2, 3, 4 and 5, L and E, which may be identical or different, are in each case, independently of one another, a bivalent radical from the group formed by -Phe-, -Cyc-, -Phe-Phe-, -Phe-Cyc-, -Cyc-Cyc-, -Pyr-, -Dio-, -G-Phe- and -G-Cyc- and their mirror images, where Phe is unsubstituted or fluorine-substituted 1,4-phenylene, Cyc is trans-1,4-cyciohexylene or 1,4-cyclohexylene, Pyr is pyrimidine-2,5-diyl or pyridine-2,5-diyl, Dio is 1,3-dioxane-2,5-diyl and G is 2-(trans-1,4-cyciohexyl)ethyl, pyrimidine-2,5-diyl, pyridine-2,5-diyl or 1,3-dioxane-2,5-diyl.

One of the radicals L and E is preferably Cyc, Phe or Pyr. E is preferably Cyc, Phe or Phe-Cyc. The media according to the invention preferably comprise one or more components selected from the compounds of the formulae 1, 2, 3, 4 and 5 in which L and E are selected from the group consisting of Cyc, Phe and Pyr and simultaneously one or more components selected from the compounds of the formulae 1, 2, 3, 4 and 5 in which one of the radicals L and E is selected from the group consisting of Cyc, Phe and Pyr and the other radical is selected from the group consisting of -Phe-Phe-, -Phe-Cyc-, -Cyc-Cyc-, -G-Phe- and -G-Cyc-, and optionally one or more components selected from the compounds of the formulae 1, 2, 3, 4 and 5 in which the radicals L and E are selected from the group consisting of -Phe-Cyc-, -Cyc-Cyc-, -G-Phe- and -G-Cyc-.

In a smaller sub-group of the compounds of the formulae 1, 2, 3, 4 and 5, R' and R" are each independently of one another, alkyl, alkenyl, alkoxy. alkoxyalkyl, alkenyloxy or alkanoyloxy having up to 8 carbon atoms. This smaller sub-group is called group A below, and the compounds are denoted by the sub-formulae 1a, 2a, 3a, 4a and 5a. In most of these compounds, R' and R" are different from one another, one of these radicals usually being alkyl, alkenyl, alkoxy or alkoxyalkyl.

In another smaller sub-group of the compounds of the formulae 1, 2, 3, 4 and 5, which is called group B, R" is -F, -Cl, -NCS or -(O)ᵢCH₃₋₍ₖ₊ₗ₎FₖClₗ, where i is 0 or 1, and k and l are 1, 2 or 3; the compounds in which R" has this meaning are denoted by the sub-formulae 1b, 2b, 3b, 4b and 5b. Particular preference is given to those compounds of the sub-formulae 1b, 2b, 3b, 4b and 5b in which R" is -F, -Cl, -NCS, -CF₃, -OCHF₂ or -OCF₃.

In the compounds of the sub-formulae 1b, 2b, 3b, 4b and 5b, R' is as defined for the compounds of the sub-formulae 1a-5a and is preferably alkyl, alkenyl, alkoxy or alkoxyalkyl.

In a further smaller sub-group of the compounds of the formulae 1, 2, 3, 4 and 5, R" is -CN; this sub-group is called group C below, and the compounds of this sub-group are correspondingly described by sub-formulae 1c, 2c, 3c, 4c and 5c. In the compounds of the sub-formulae 1c, 2c, 3c, 4c and 5c. R' is as defined for the compounds of the sub-formulae 1a-5a and is preferably alkyl, alkoxy or alkenyl.

In addition to the preferred compounds of groups A, B and C, other compounds of the formulae 1, 2, 3, 4 and 5 having other variants of the proposed substituents are also customary. All these substances can be obtained by methods which are known from the literature or analogously thereto.

Besides compounds of the formula I according to the invention, the media according to the invention preferably comprise one or more compounds selected from group A and/or group B and/or group C. The proportions by weight of the compounds from these groups in the media according to the invention are preferably:

| | |
|---|---|
| Group A | 0 to 90%, preferably 20 to 90%, in particular 30 to 90% |
| Group B | 0 to 80%, preferably 10 to 80%, in particular 10 to 65% |
| Group C | 0 to 80%, preferably 5 to 80%, in particular 5 to 50%, |

the sum of the proportions by weight of the group A and/or B and/or C compounds present in the particular media according to the invention preferably being 5%-90% and in particular from 10% to 90%.

The media according to the invention preferably comprise from 1 to 40%, particularly preferably from 5 to 30%. of the compounds according to the invention. Further preferred media are those which comprise more than 40%, in particular from 45 to 90%, of compounds according to the invention. The media preferably comprise three, four or five compounds according to the invention.

The media according to the invention are prepared in a manner which is customary per se. In general, the components are dissolved in one another, advantageously at elevated temperature. By means of suitable additives, the liquid-crystalline phases can be modified in accordance with the invention in such a manner that they can be used in all types of liquid-crystal display elements which have been disclosed hitherto. Additives of this type are known to those skilled in the art and are described in detail in the literature (H. Kelker/R Hatz, Handbook of Liquid Crystals, Verlag Chemie, Weinheim, 1980). For example, pleochroic dyes can be added for the production of coloured guest-host systems, or substances can be added to modify the dielectric anisotropy, the viscosity and/or the alignment of the nematic phases.

The examples below are intended to illustrate the invention without representing a limitation. Above and below, percentages are percent by weight. All temperatures are given in degrees Celsius. m.p. denotes melting point, cl.p. = clearing point, Tg = glass transition temperature. Furthermore. C = crystalline state, N = nematic phase, Sm = smectic phase and I = isotropic phase. The numbers between these symbols indicate the conversion temperatures. Δn denotes the optical anisotropy (589 nm, 20°C) and Δε the dielectric anisotropy (1 kHz, 20°C). The Δn and Δε values of the compounds according to the invention were obtained by extrapolation from liquid-crystalline mixtures consisting of 10% of the particular compound according to the invention and 90% of the commercially available liquid crystal ZLI 4792 (Merck, Darmstadt).

The viscosity (mm²/sec) was determined at 20°C. "Conventional work-up" means that water is added if necessary, the mixture is extracted with methylene chloride, diethyl ether or toluene, the phases are separated, the organic phase is dried and evaporated, and the product is purified by distillation under reduced pressure or crystallization and/or chromatography.

The following abbreviations are used:

| | |
|---|---|
| THF | tetrahydrofuran |
| KOtBu | potassium tert-butoxide |
| RT | room temperature |
| MTB | ether methyl tert-butyl ether |
| DCC | N,N-dicyclohexylcarbodiimide |
| DMAP | 4-dimethylaminopyridine |

### Example 1

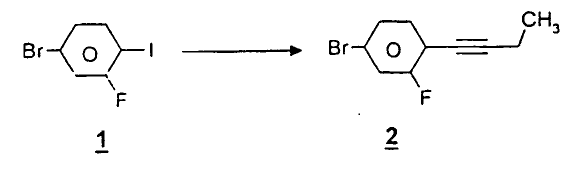

11.4 g of 1-butyne were fed into 100 ml of triethylamine, and 60.2 g of 1-bromo-3-fluoro-4-iodobenzene (**1**)and 300 ml of triethylamine were added to the resultant mixture at 10°C with stirring. A mixture of 2.8 g of Pd(II) catalyst and 0.4 g of Cul were subsequently added. The reaction mixture was stirred overnight at RT and subsequently subjected to conventional work-up, giving **2**.

### Example 2

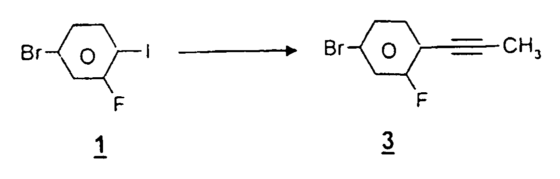

Firstly, 7.59 g of propyne were fed at 10°C into a solution of 50.0 g of 1-bromo-3-fluoro-4-iodobenzene (**1**) in 315 ml of triethylamine and 235 ml of THF, and, after the cooling had been removed, 2.216 g of bis(triphenylphosphine)Pd(II) were added. After the mixture had been stirred overnight, it was subjected to conventional work-up, giving **3**.

### Example 3

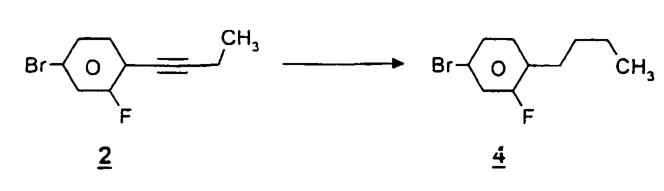

45.8 g of **2**were hydrogenated in 460 ml of hexane in the presence of 4.6 g of Pt/C (10%) at RT and a pressure of 3.1 bar, and subjected to conventional work-up, giving **4**.

### Example 4

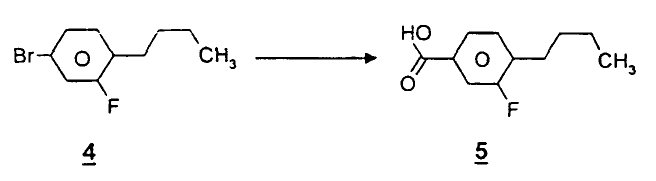

83.92 ml of a 15% solution of butyllithium in hexane were added dropwise at -70°C to a solution of 32.0 g of **4** in 270 ml of THF. After the mixture had been stirred for 30 minutes, an excess of carbon dioxide was passed into the solution in such a way that the temperature did not rise above -55°C. The cooling was subsequently removed, and the mixture was subjected to conventional work-up, giving **5**.

### Example 5

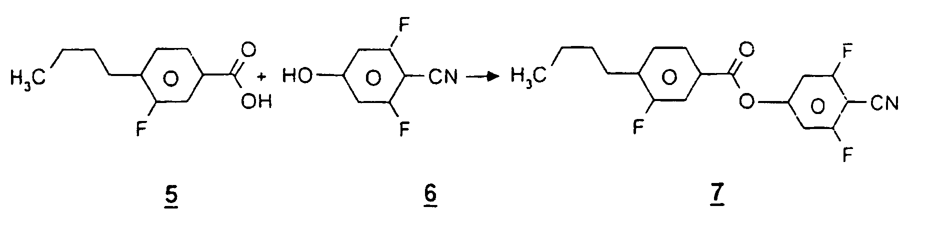

3.40 g of **5** were added to a solution of 2.40 g of **6**, 3.40 g of DCC and 0.400 g of DMAP in 30 ml of toluene in such a way that the temperature did not rise above 40°C. After the mixture had been stirred overnight, 0.40 g of oxalic acid dihydrate were added to the reaction mixture. The mixture was stirred for 1 hour and subsequently filtered. The filtrate was subjected to conventional work-up, giving **7** (C 60 I).

### Example 6

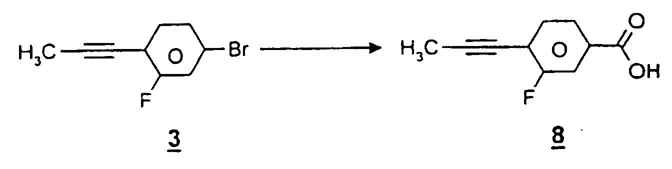

53.08 ml of a 15% solution of butyllithium in hexane were added dropwise at -65°C to a solution of 18.60 g of **3** in 112 ml of THF. An excess of carbon dioxide was subsequently passed into the solution in such a way that the temperature did not rise above -50°C. The cooling was subsequently removed, and the mixture was subjected to conventional work-up, giving **8**.

### Example 7

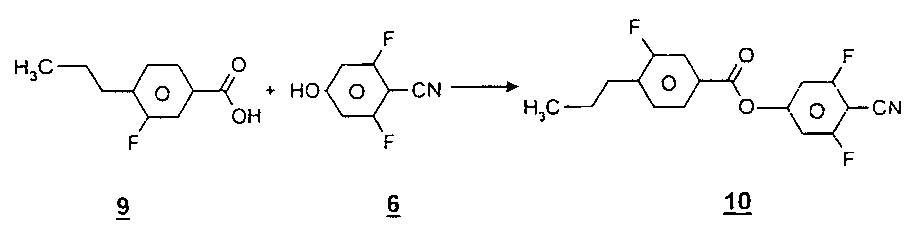

2.50 g of **9** (obtainable from **8** by hydrogenation as described in Example 3) were added to a solution of 2.17 g of **6**, 2.89 g of DCC and 0.342 g of DMAP in 30 ml of toluene in such a way that the temperature did not rise above 40°C. After the mixture had been stirred overnight, 0.353 g of oxalic acid dihydrate were added to the reaction mixture. The mixture was stirred for 1 hour and subsequently filtered. The filtrate was subjected to conventional work-up, giving **10** (C 70 I).

### Example 8

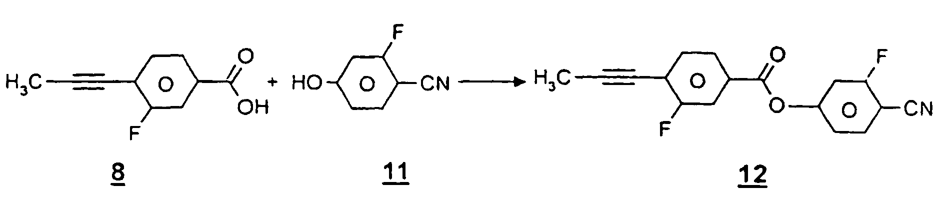

A mixture of 1.00 g of **8**, 0.739 g of **11**, 1.17 g of DCC and 0.115 g of DMAP in 11 ml of toluene was stirred at RT for 2 days. After 0.136 g had been added, the reaction mixture was stirred for 30 minutes and subsequently filtered. The filtrate was subjected to conventional work-up, giving **12** (C 140 N (85.1) I).

### Example 9

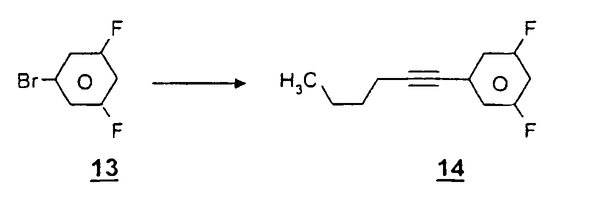

A mixture of 40.00 g of **13,** 30.50 ml of 1-hexyne, 5.988 g of tetrakis(triphenylphosphine)palladium and 300 ml of pyrrolidine was stirred at RT for 2 hours. The reaction mixture was subjected to conventional work-up, giving **14.**

### Example 10

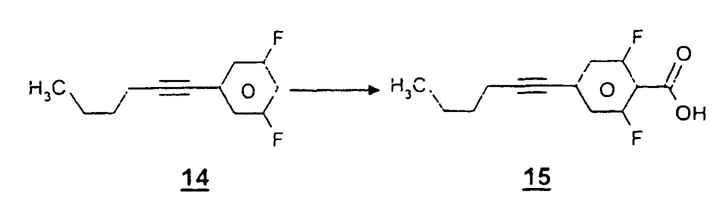

59.72 ml of a 15% solution of butyllithium in hexane were added dropwise at -70°C to a solution of 19.10 g of **14** in 100 mi of THF. The mixture was stirred for 30 minutes. An excess of carbon dioxide was subsequently passed into the solution in such a way that the temperature did not rise above -55°C. The cooling was subsequently removed, and the mixture was subjected to conventional work-up, giving **15**.

### Example 11 - Comparison Example

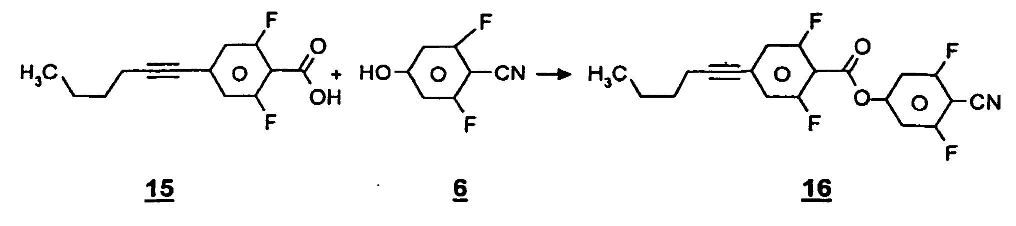

A solution of 9.483 g of DCC in 30 ml of dichloromethane was added dropwise at RT to a mixture of 7.30 g of **15,** 4.99 g of 2,6-difluoro-4-hydroxybenzonitrile (6) and 0.749 g of DMAP in 70 ml of dichloromethane. After the reaction mixture had been stirred overnight, 1.931 g of oxalic acid dihydrate were added. The mixture was stirred for 1 hour and subsequently filtered. The filtrate was subjected to conventional work-up, giving **16** (C 47 I, Δε = 59.6, Δn = 0.141).

### Example 12

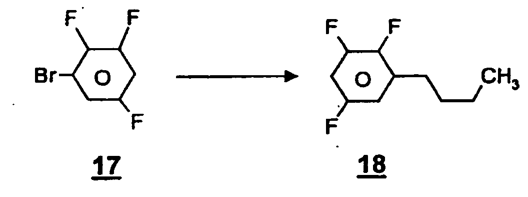

79.52 ml of a 15% solution of butyllithium in hexane were added dropwise at RT to a solution of 17.762 g of zinc chloride in 260 ml of THF. After 3.468 g of PdCl₂-dppf had been added, the mixture was heated to the boil, and 50.00 g of **17** were added dropwise. The reaction mixture was refluxed overnight and subsequently subjected to conventional work-up, giving **18**.

### Example 13

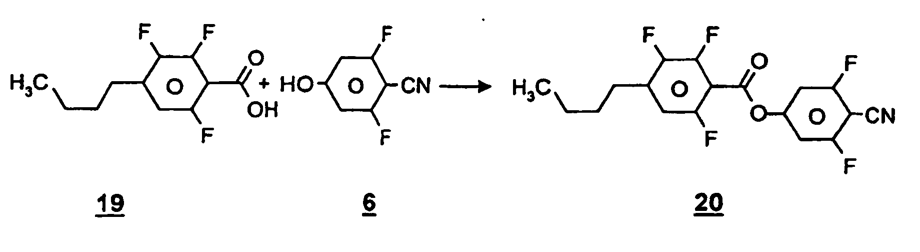

**19** (obtainable from **18** analogously ro Example 10) is reacted with 2,6-difluoro-4-hydroxybenzonitrile (**6**) in accordance with Example 11 to give **20** (C 56 I, Δε = 39.0, Δn = 0.089).

The following examples can be prepared analogously:

### Examples 14 - 172 (Examples 113 - 152 are Comparison Examples

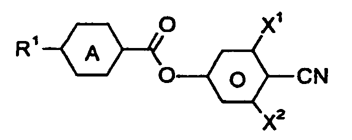

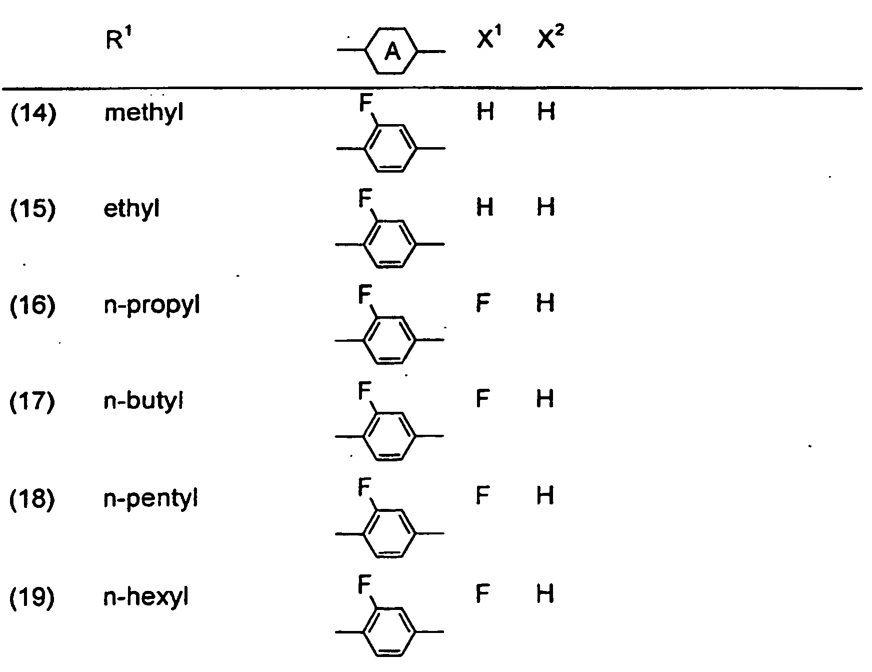

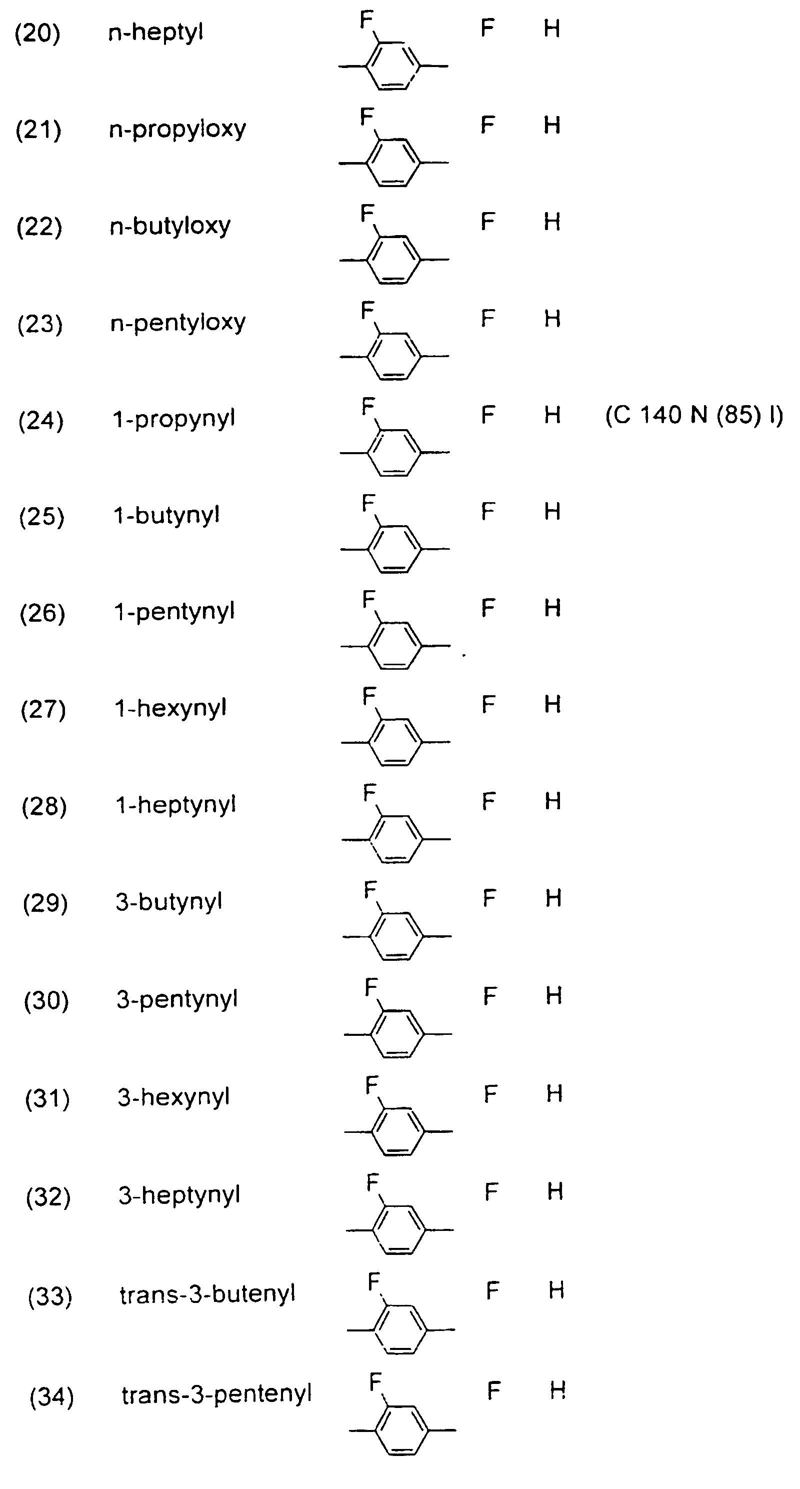

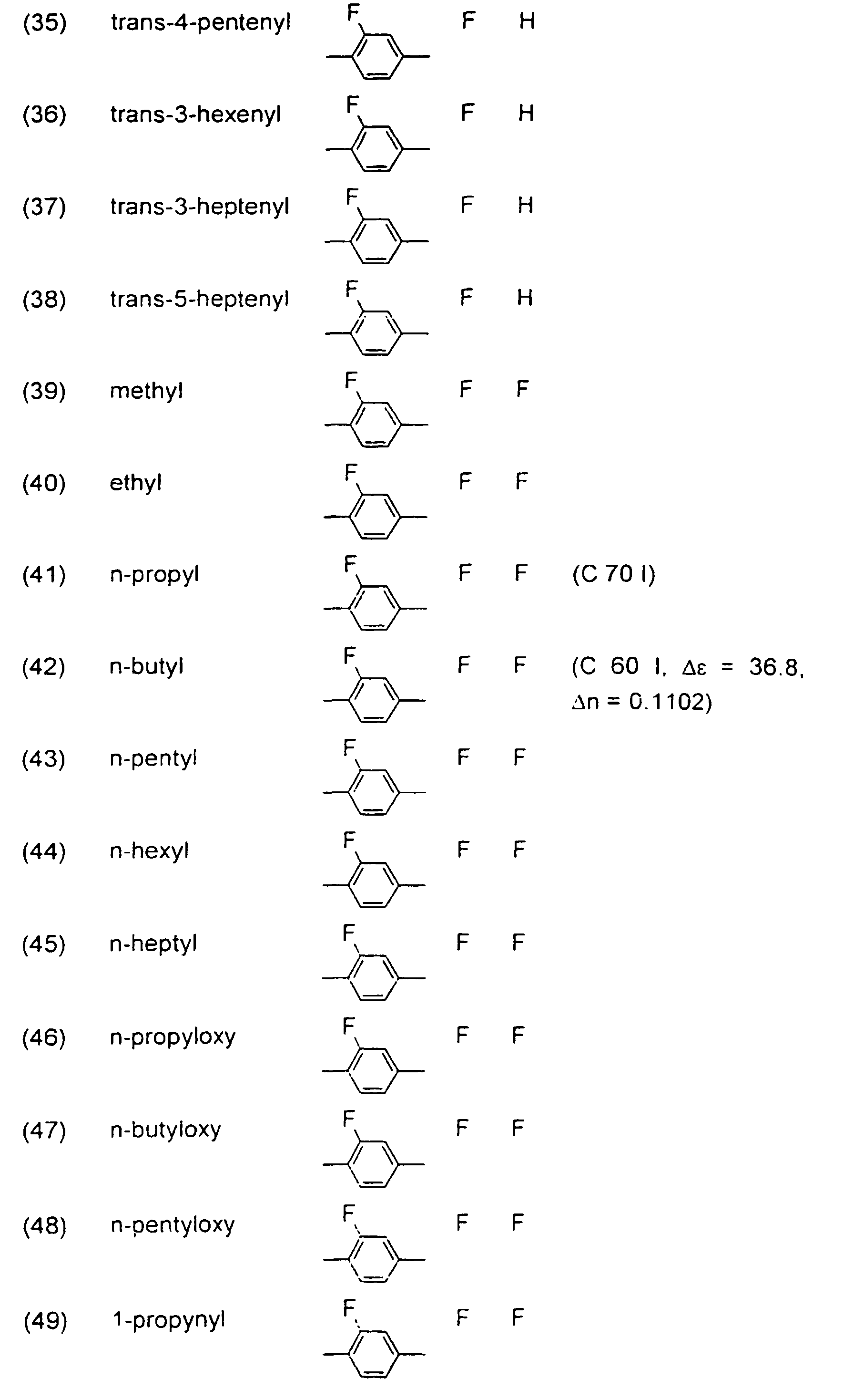

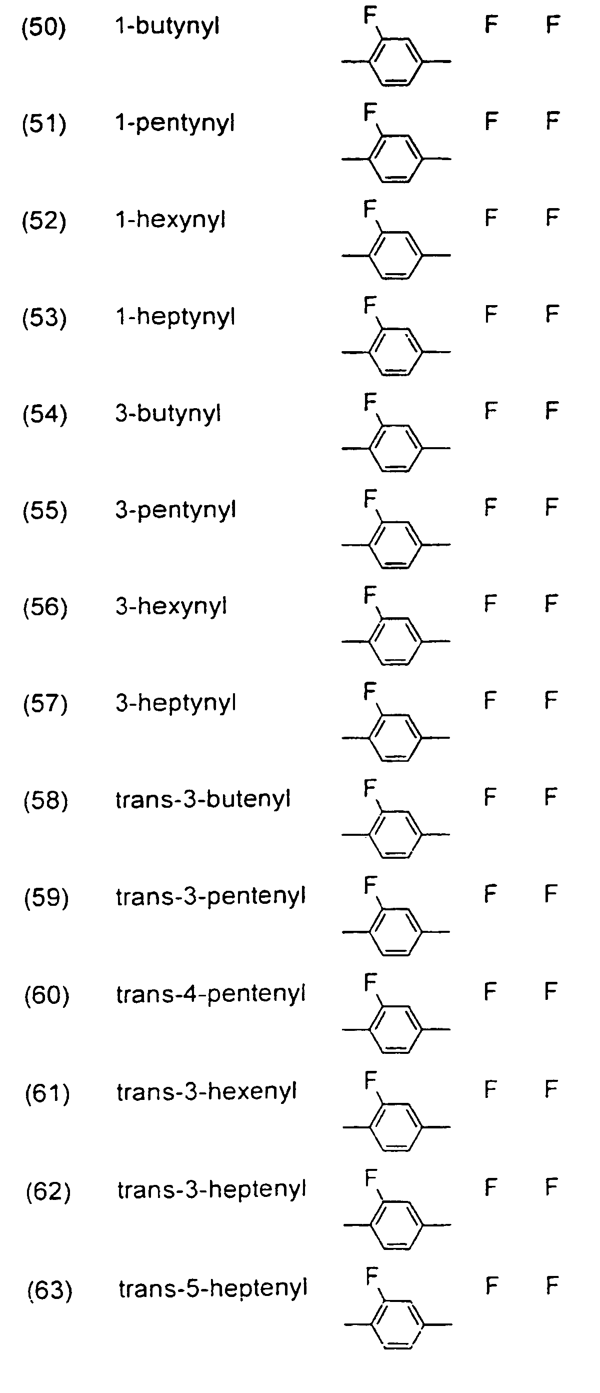

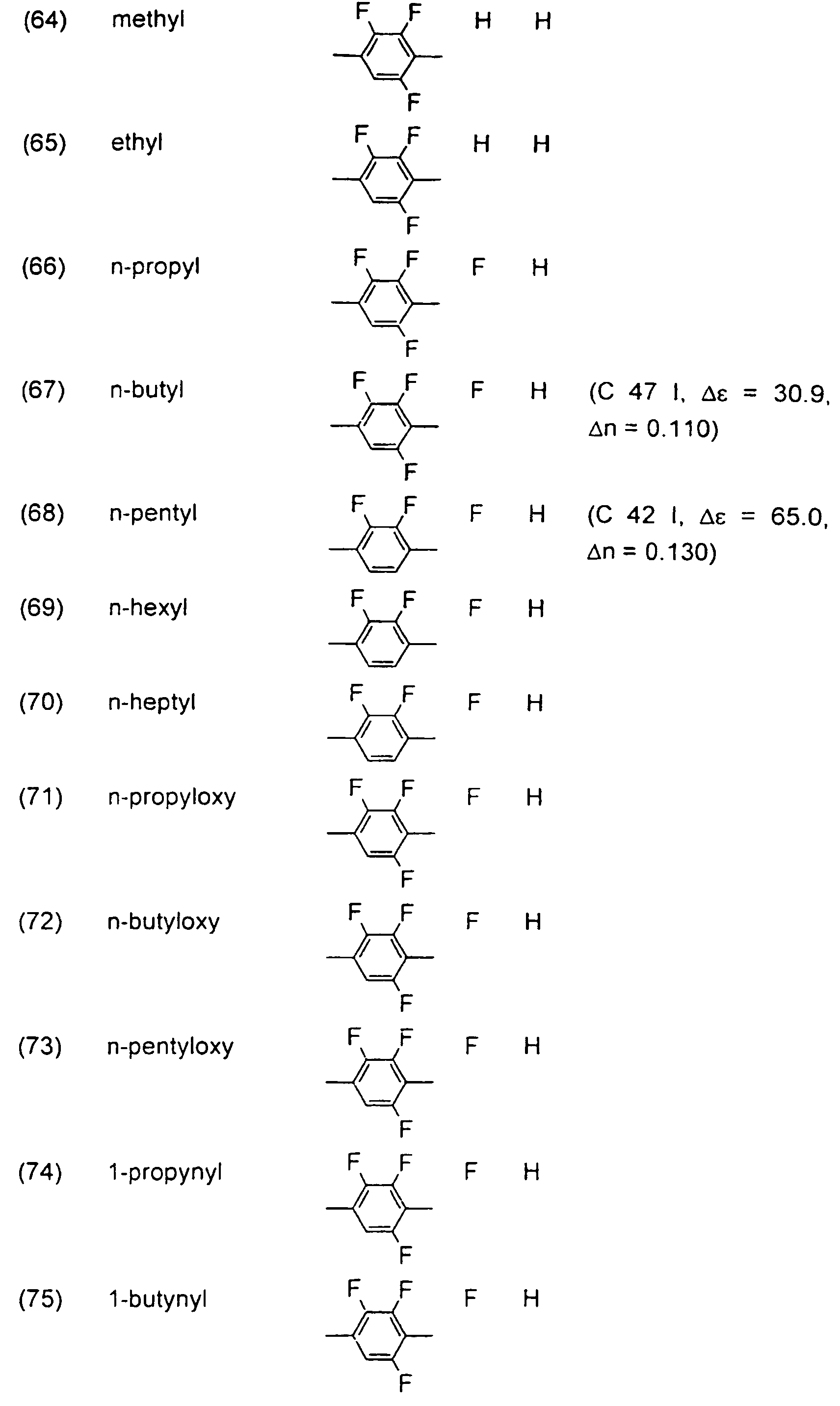

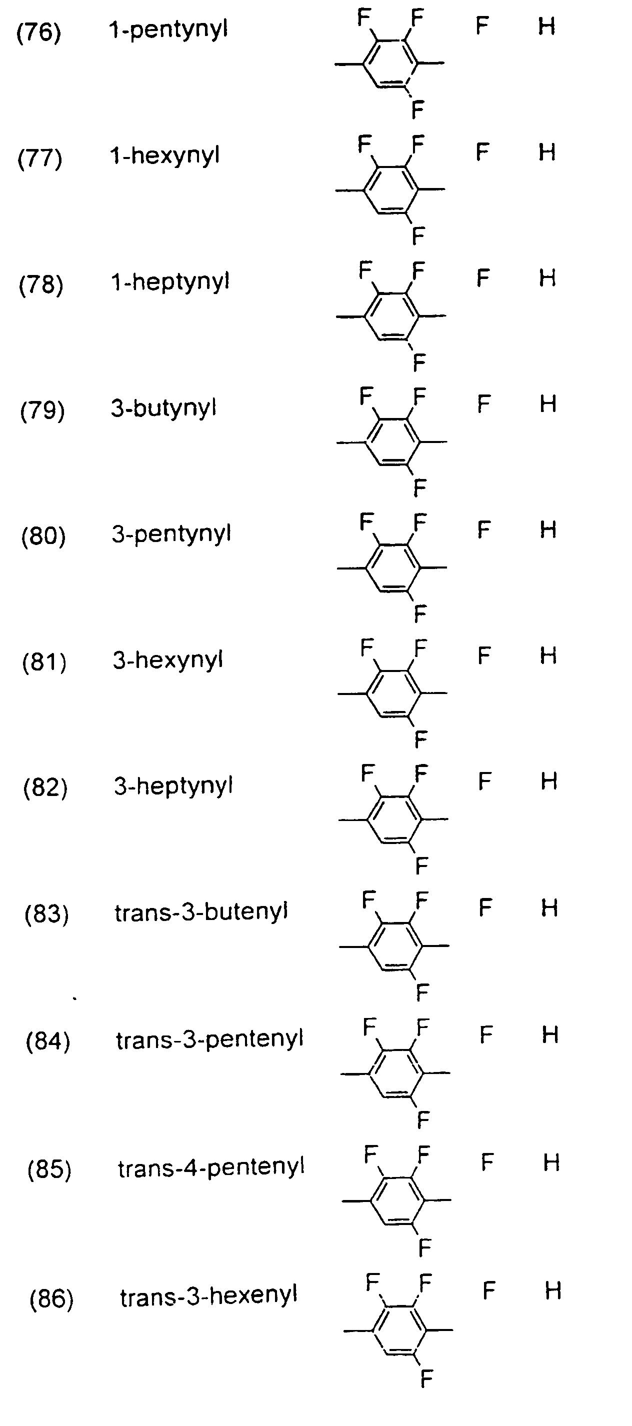

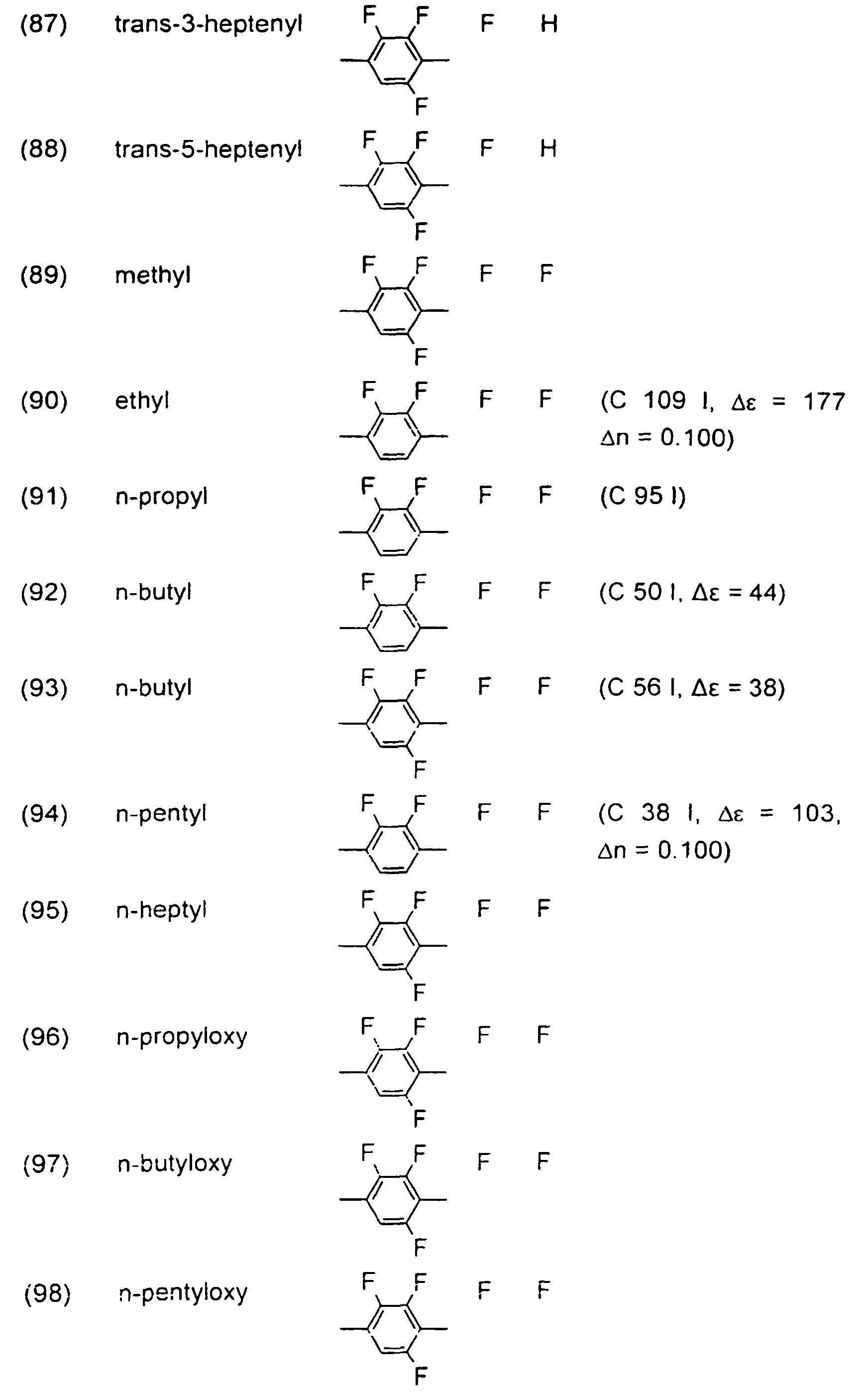

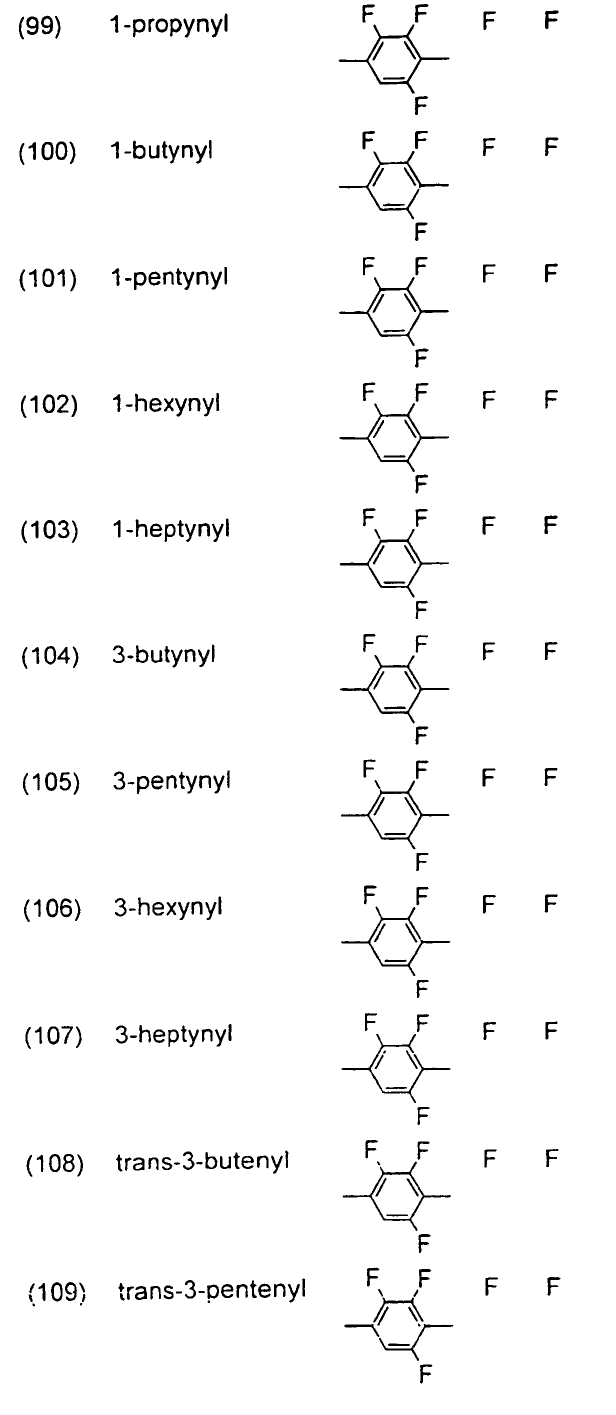

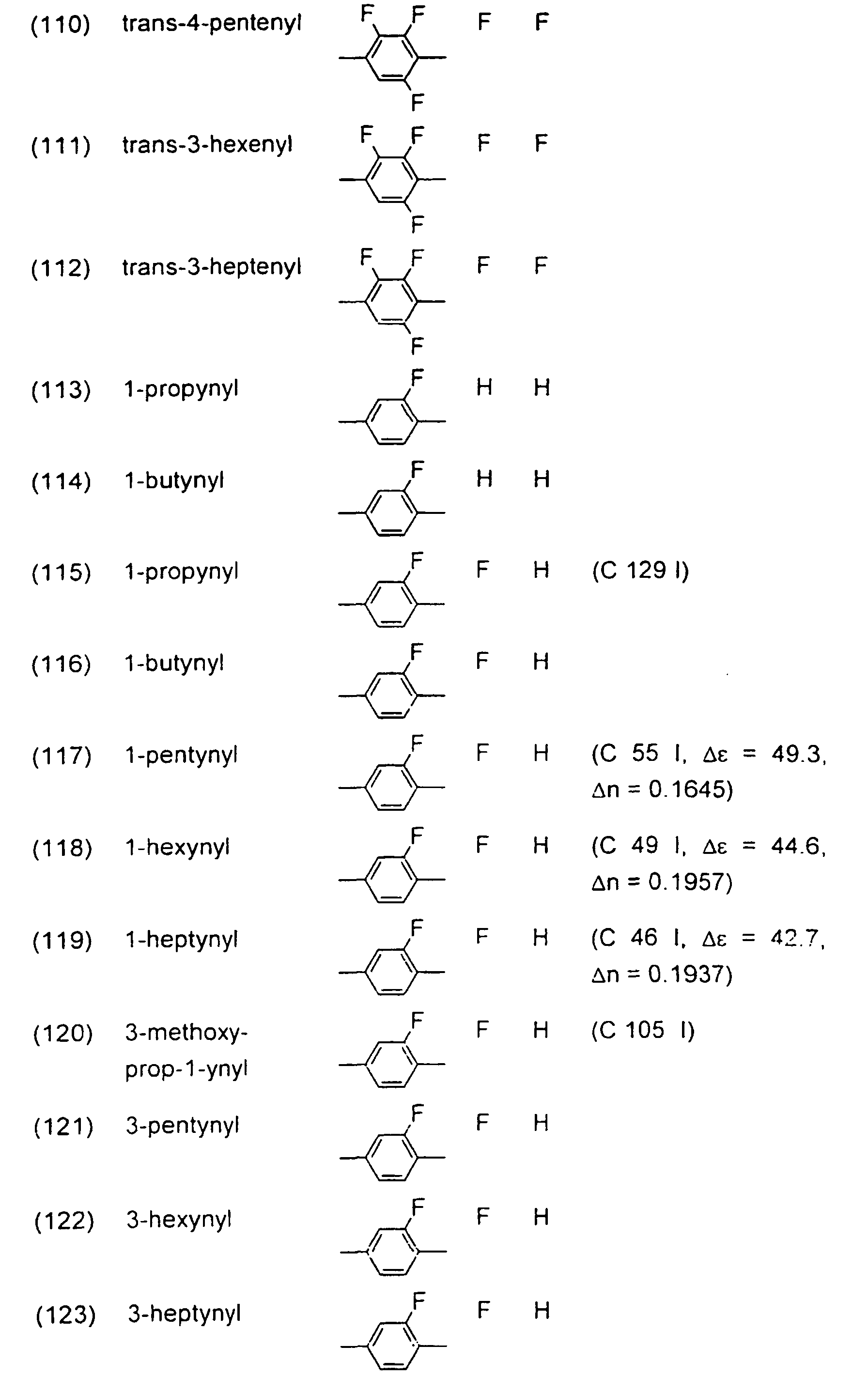

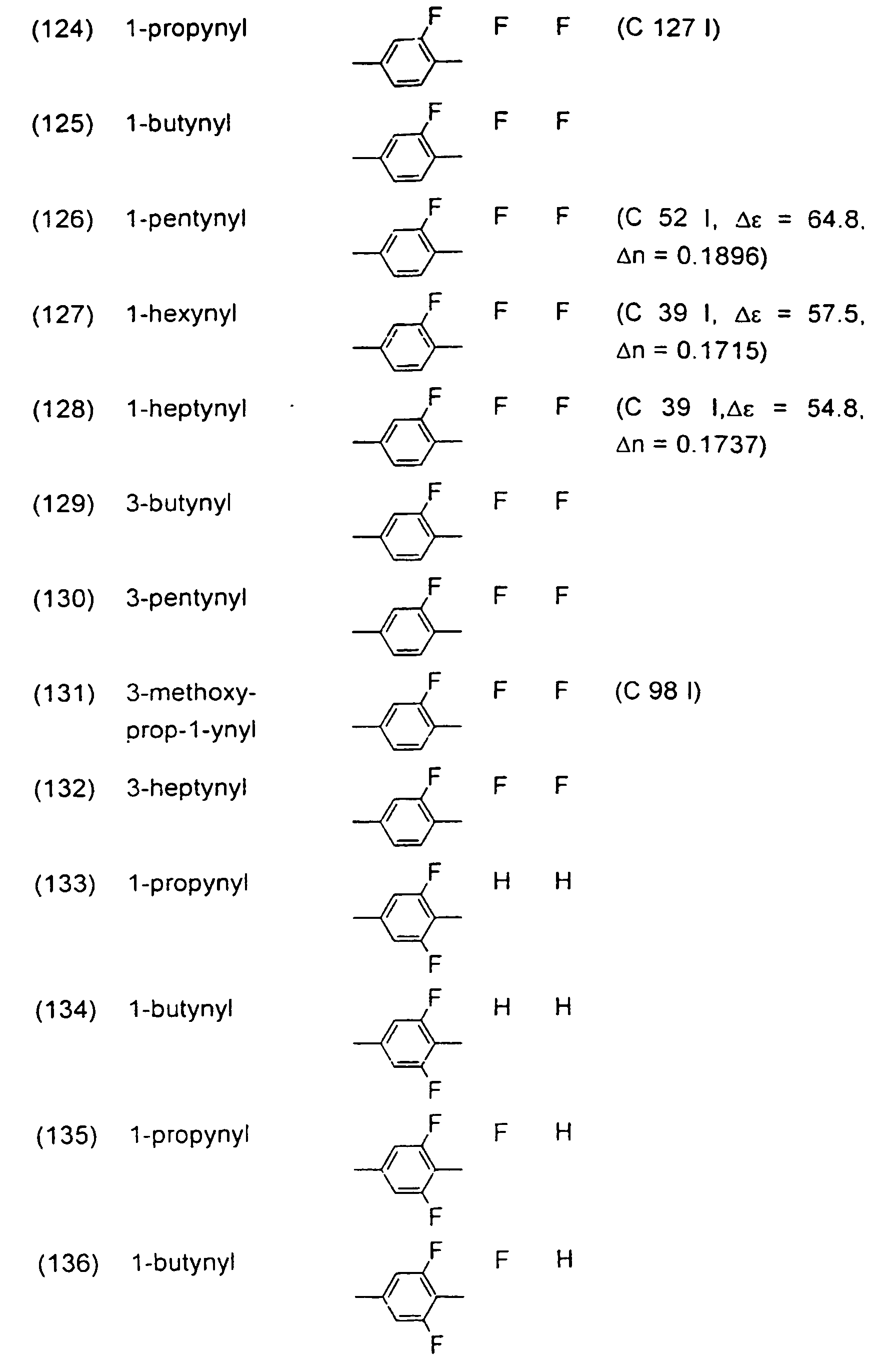

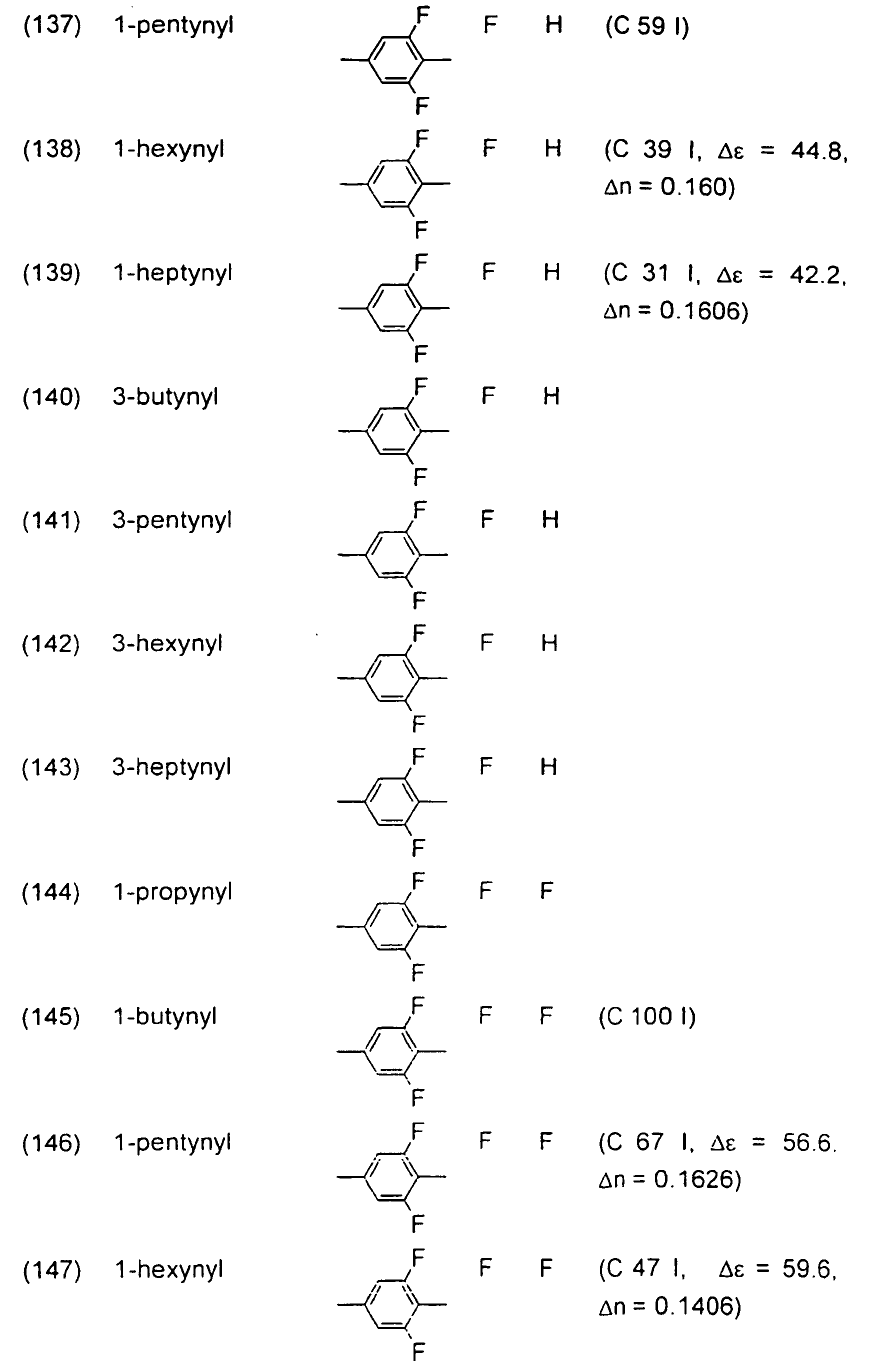

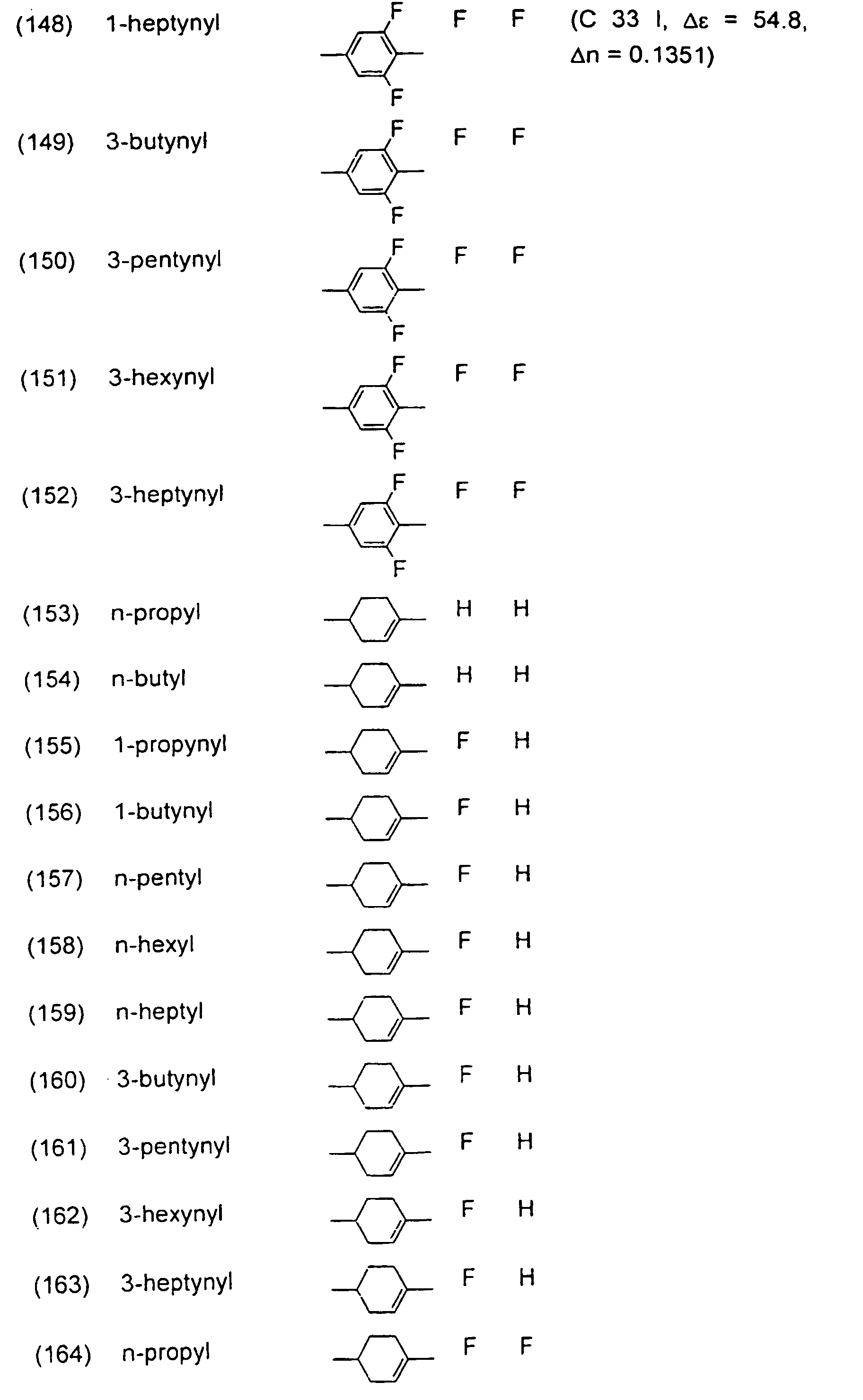

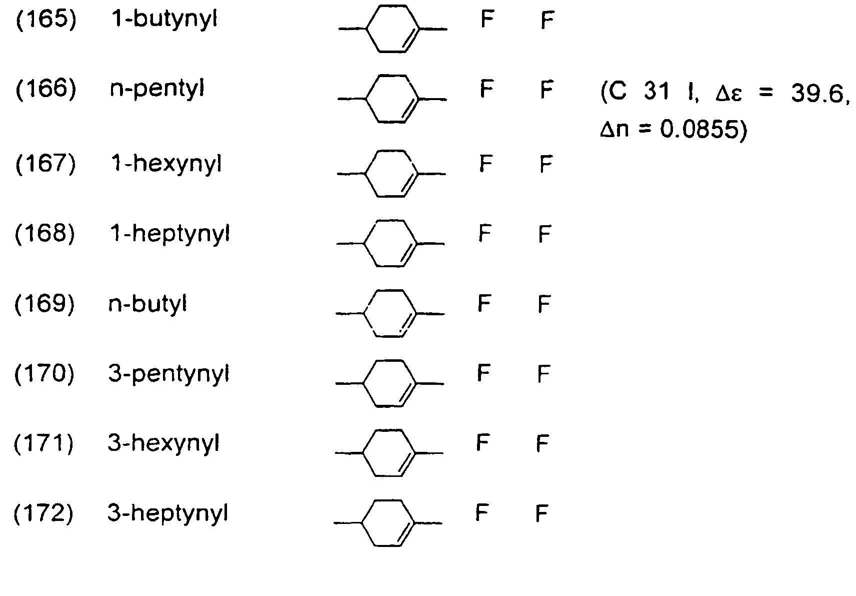

### Examples 173- 182

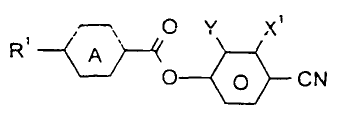

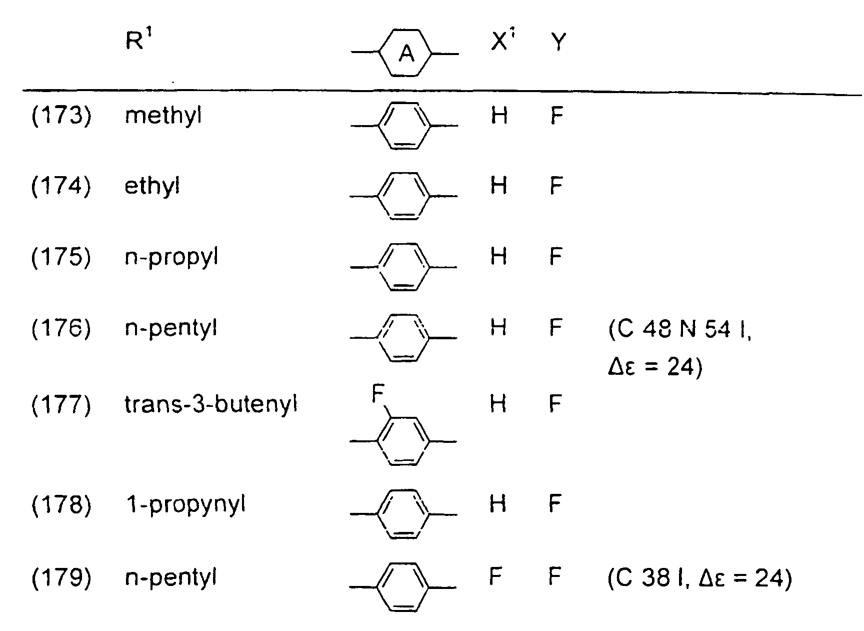

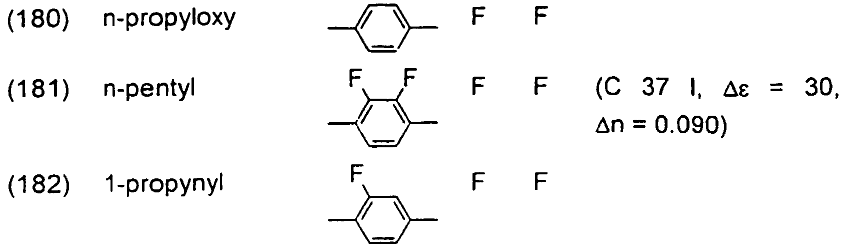

### Examples 183 - 192

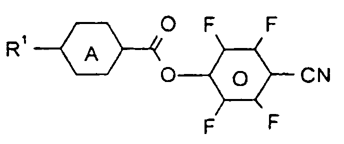

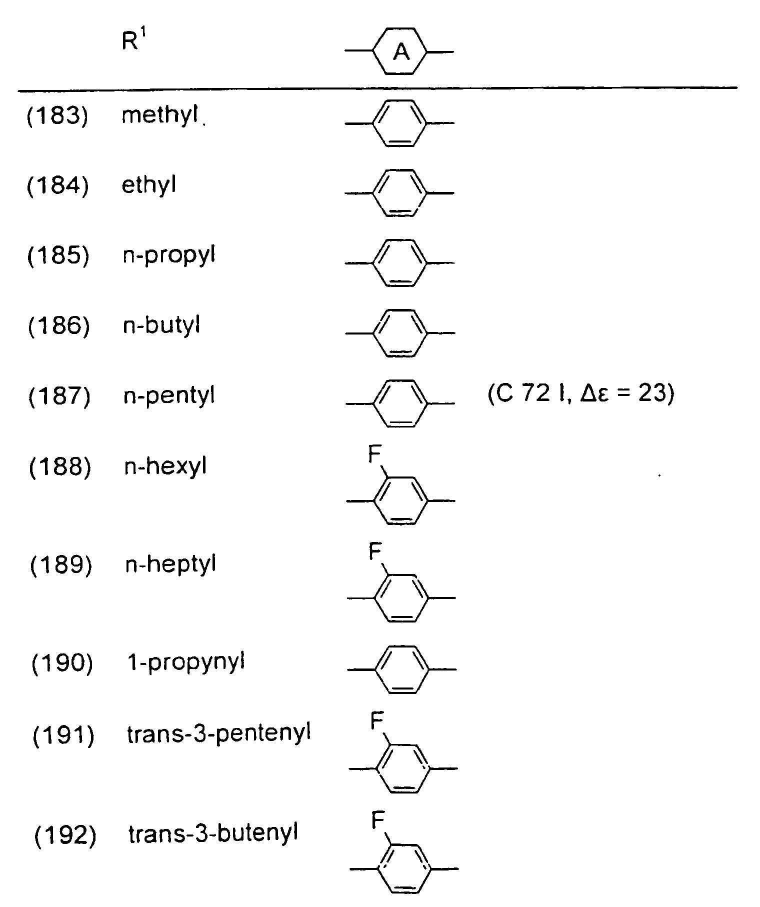

### Examples 193 - 233

### (Examples 193-192, 204-208 and 211-233 are Comparison Examples)

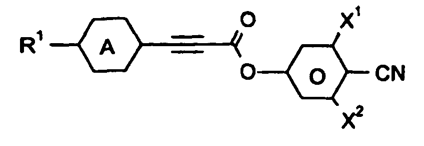

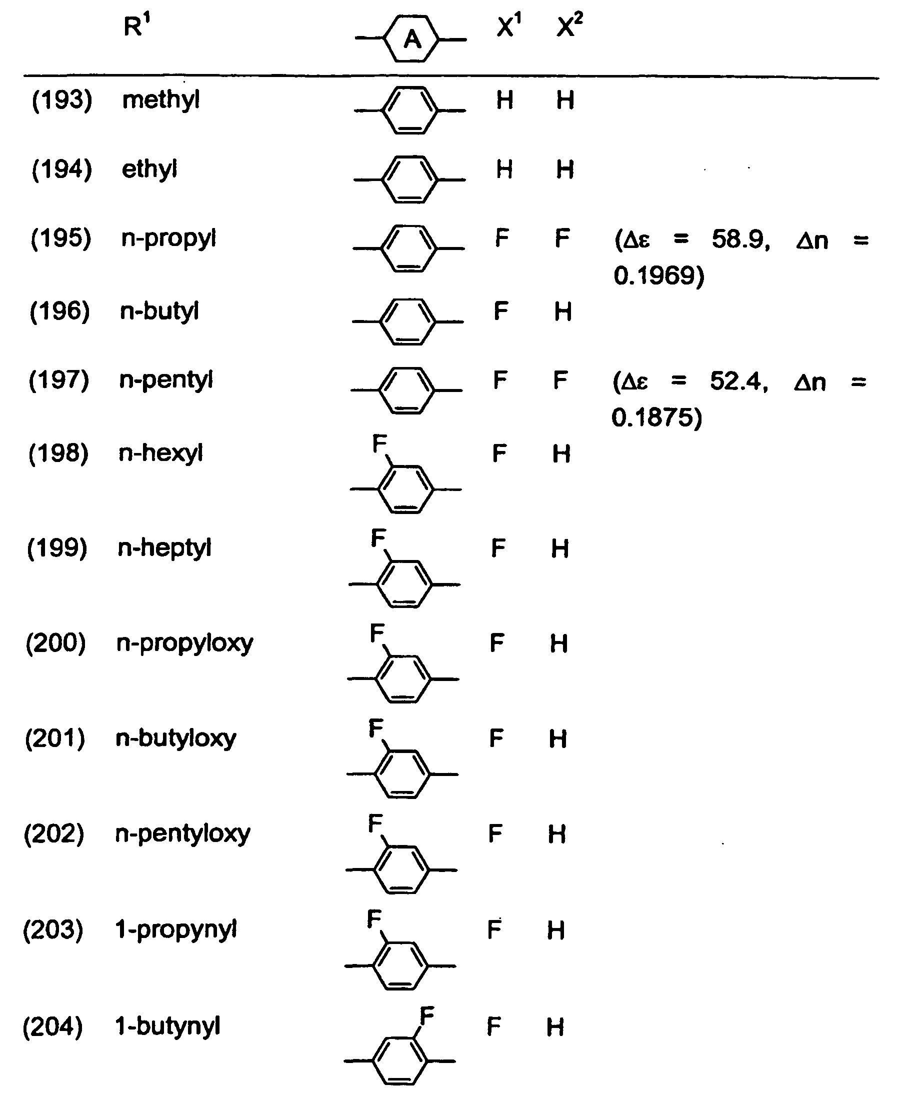

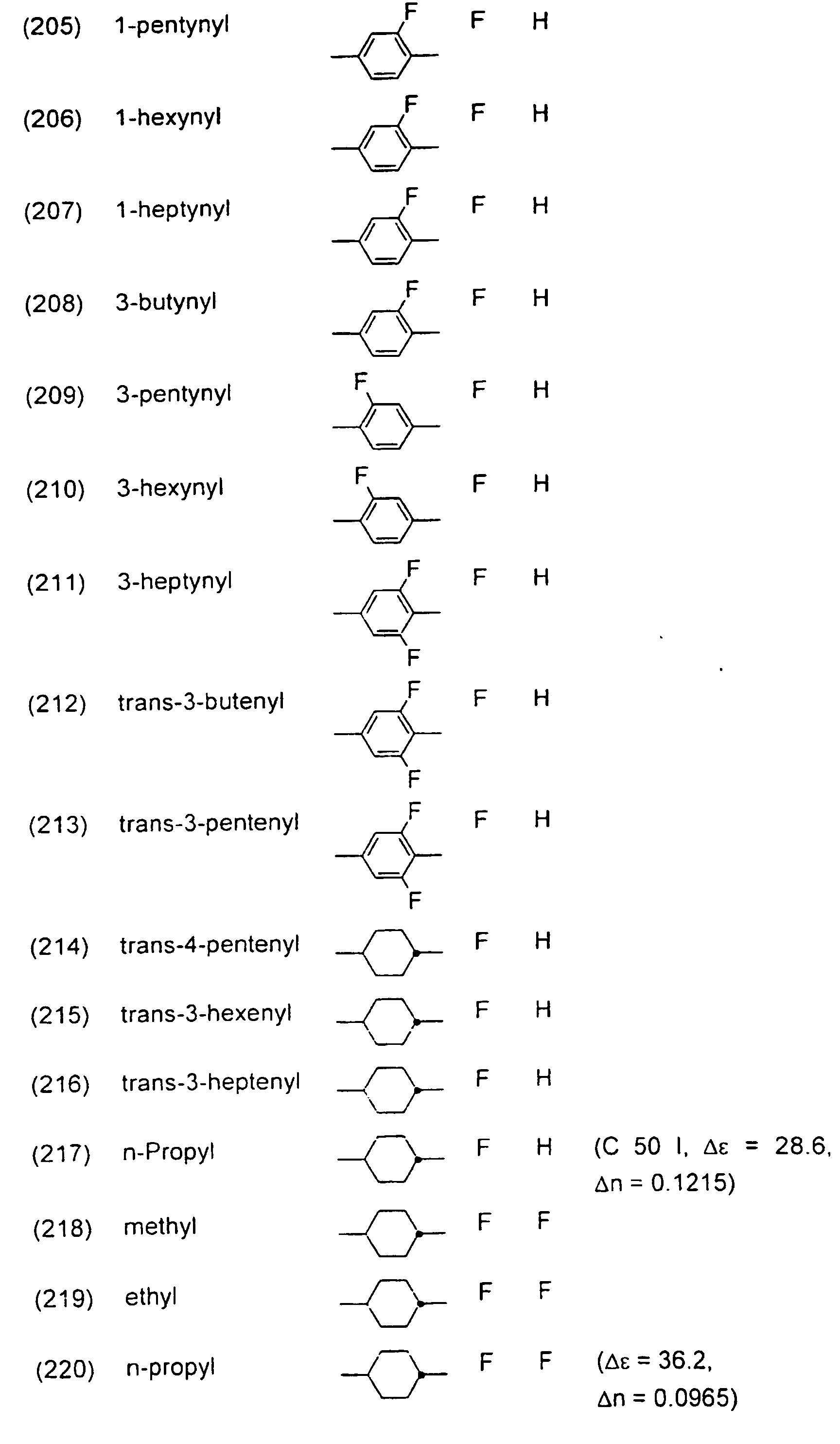

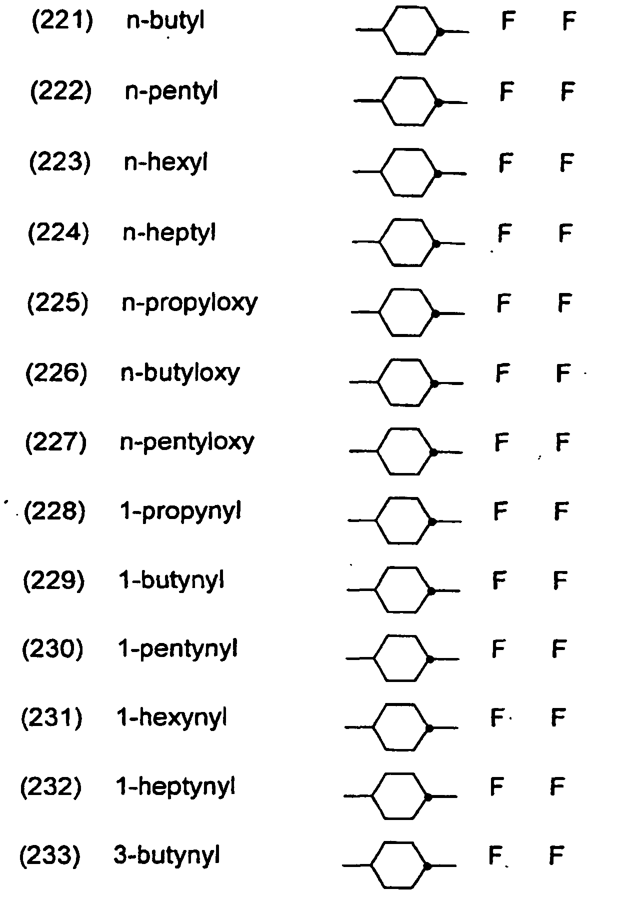

### Examples 234 - 253 (Comparison Examples)

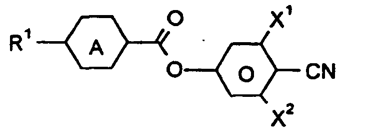

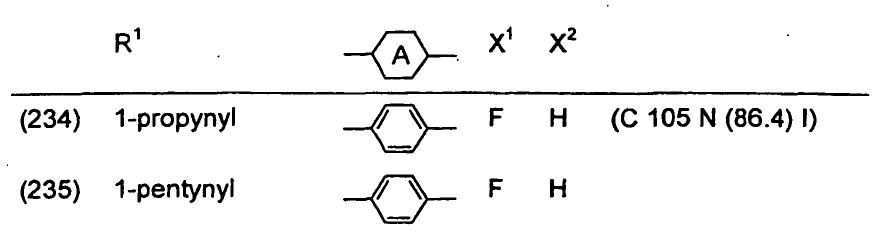

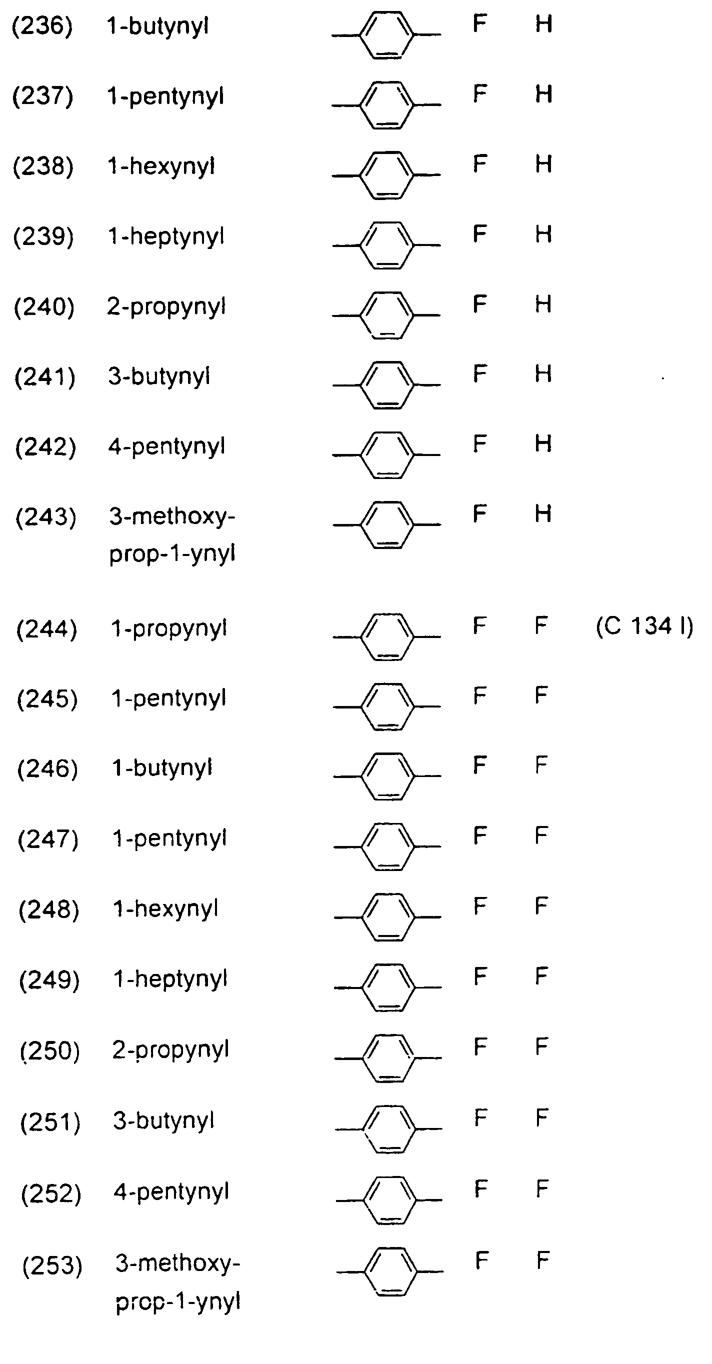

## Claims

1. Benzoic acid esters of the formula I 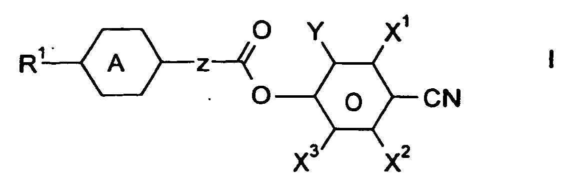 in which
R¹ is H, an alkyl radical having 1-12 carbon atoms which is unsubstituted or at least monosubstituted by halogen or CN and in which, in addition, one or more CH₂ groups may each, independently of one another, be replaced by -O-, -C≡C- or -CH=CH- in such a way that heteroatoms are not connected directly, 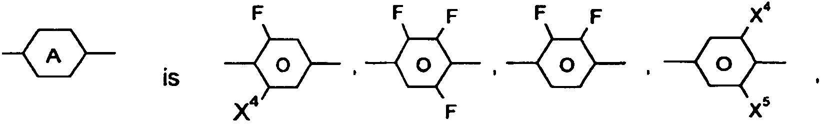 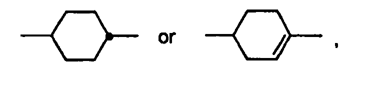
Z is -C≡C- or a single bond,
Y is H or F,
and
X¹, X², X³, X⁴ and X⁵, independently of one another, are H or F, with the proviso that, in the compounds of the formula I in which 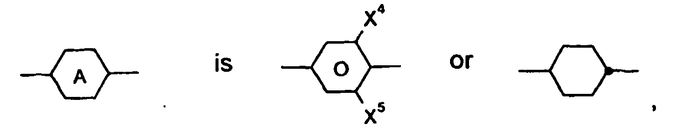
Y is F
and
a)
R¹ is an alkyl, oxaalkyl or alkoxy radical having 1 to 10 carbon atoms in which one or more CH₂ groups have been replaced by -C≡C-, and/or
b)
Z is -C≡C-.

2. Benzoic acid esters according to Claim 1, **characterized in that** R¹ is an alkyl, oxaalkyl or alkoxy radical having 1 to 10 carbon atoms in which one or more CH₂ groups have been replaced by -C≡C-.

3. Benzoic acid esters according to Claim 1 or 2, in which one of the two radicals X¹ and X² is H and the other is F or in which the radicals are simultaneously F.

4. Benzoic acid esters according to Claim 1, 2 or 3, in which both radicals X¹ and X⁴ are simultaneously F.

5. Benzoic acid esters according to Claim 1, 2 or 3, in which the

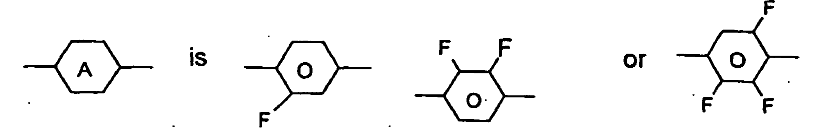

6. Benzoic acid esters according to one of the preceding Claims, in which Y is F.

7. Benzoic acid esters of the formulae I1 to I21 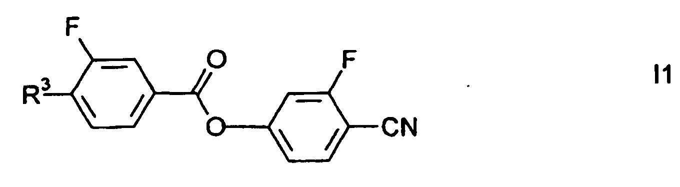 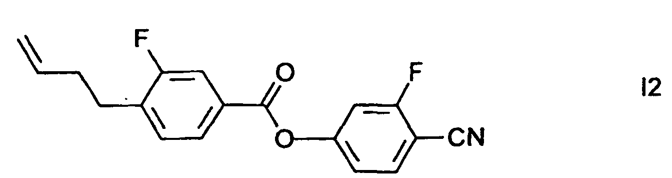 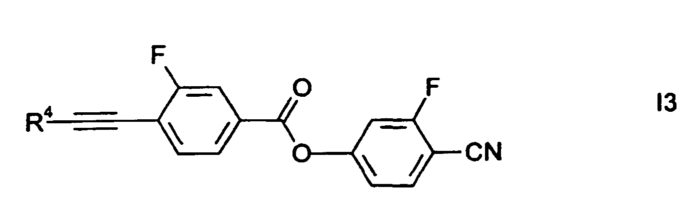 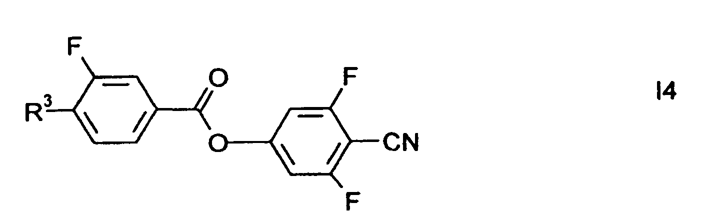 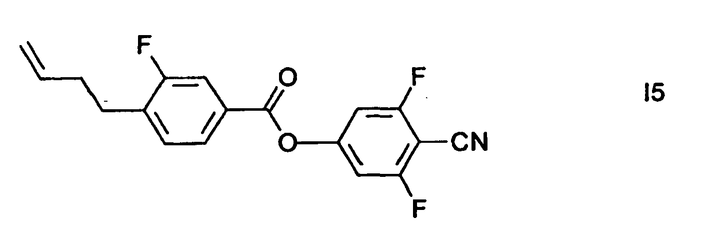 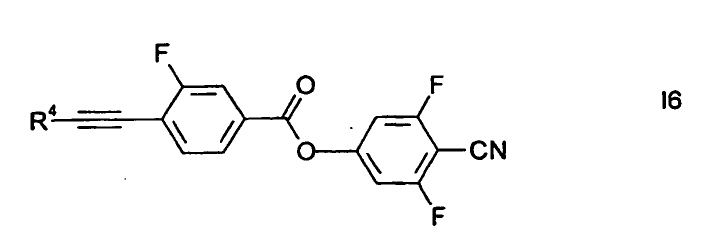 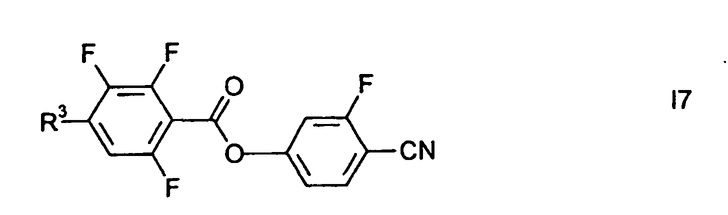 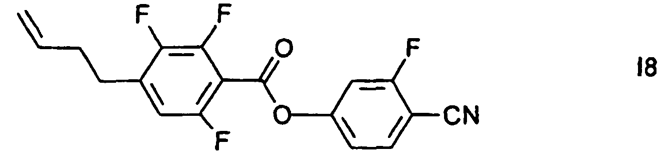 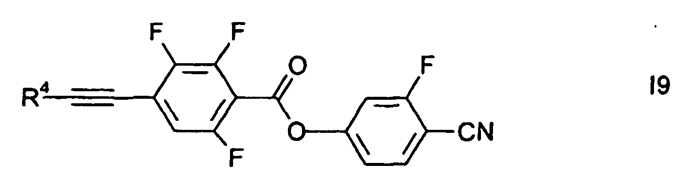 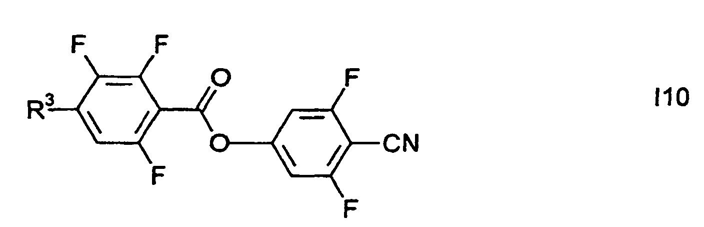 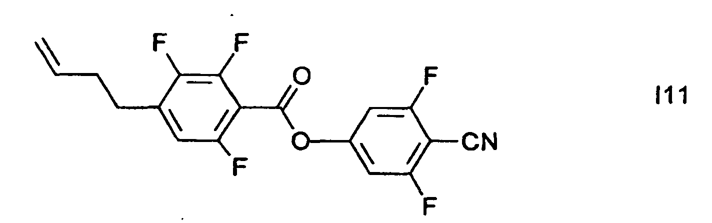 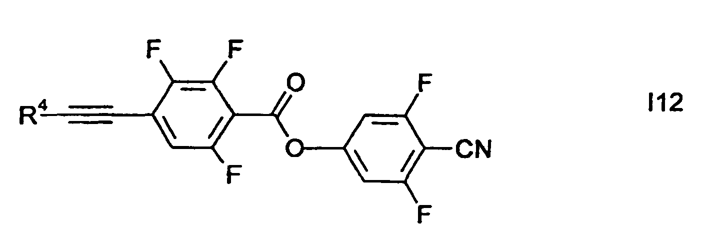 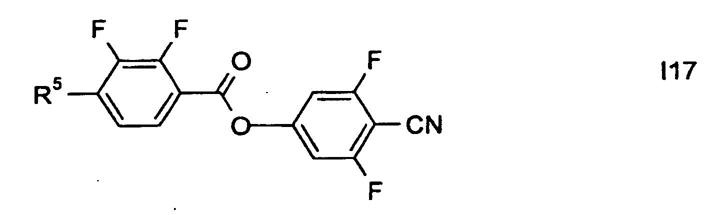 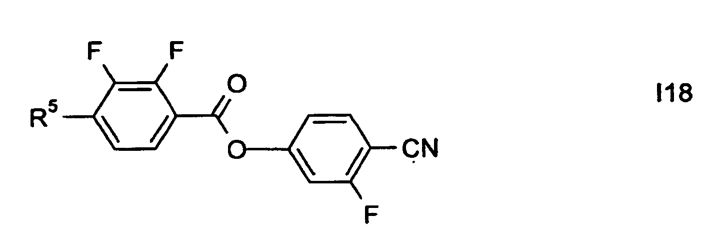 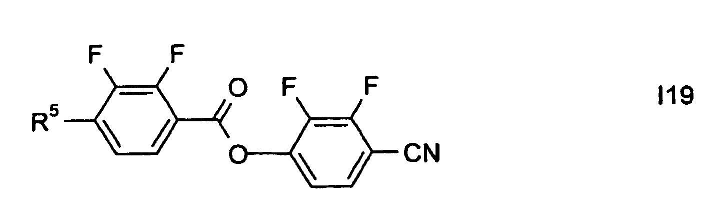 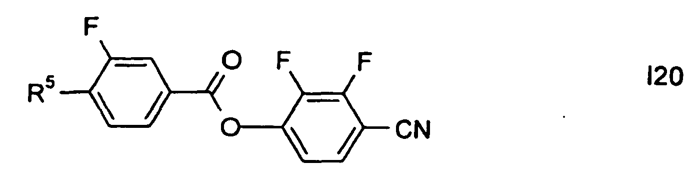 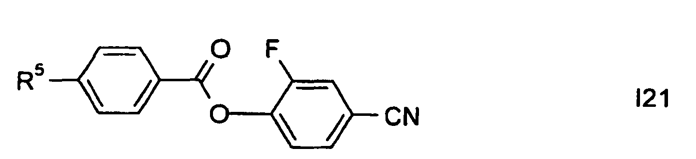 in which X² and X⁴. independently of one another, are H or F, R³ is alkyl having 1 to 10 carbon atoms, R⁴ is H or alkyl or oxaalkyl having 1 to 7 carbon atoms, and R⁵ is alkyl or alkenyl having 2 to 7 carbon atoms.

8. Use of compounds of the formula I according to Claims 1 to 7 as components of liquid crystalline media.

9. Liquid-crystalline medium having at least two liquid-crystalline components, **characterized in that** it comprises at least one compound of the formula I.

10. Liquid-crystal display element, **characterized in that** it contains a liquid-crystalline medium according to Claim 9.

11. Electro-optical display element, **characterized in that** it contains, as dielectric, a liquid-crystalline medium according to Claim 9.

## Patentansprüche

1. Benzoesäureester der Formel I 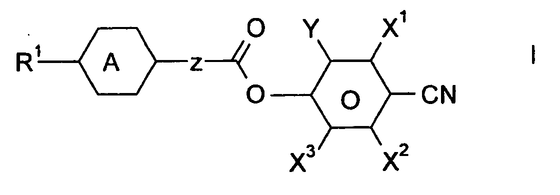 in der
R¹ H, einen Alkylrest mit 1-12 Kohlenstoffatomen bedeutet, der unsubstituiert oder mindestens einfach durch Halogen oder CN substituiert ist und in dem zusätzlich eine oder mehrere CH₂-Gruppen jeweils unabhängig voneinander durch -O-, -C≡C- oder -CH=CH- so ersetzt sein können, dass Heteroatome nicht direkt miteinander verknüpft sind, 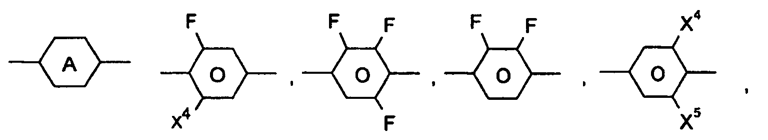 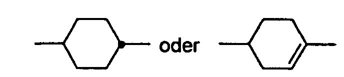
bedeutet,
Z -C≡C- oder eine Einfachbindung bedeutet,
Y H oder F bedeutet,
und
X¹, X², X³, X⁴ und X⁵ unabhängig voneinander H oder F bedeuten, mit der Maßgabe, dass in den Verbindungen der Formel I, in denen 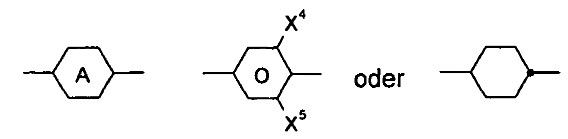 bedeutet,
Y F bedeutet
und/oder
a)
R¹ einen Alkyl-, Oxaalkyl- oder Alkoxyrest mit 1 bis 10 Kohlenstoffatomen bedeutet, in dem eine oder mehrere CH₂-Gruppen durch -C≡C- ersetzt sind, und/oder
b)
Z -C≡C- bedeutet.

2. Benzoesäureester nach Anspruch 1, **dadurch gekennzeichnet, dass** R¹ einen Alkyl-, Oxaalkyl- oder Alkoxyrest mit 1 bis 10 Kohlenstoffatomen bedeutet, in dem eine oder mehrere CH₂-Gruppen durch -C≡C- ersetzt sind.

3. Benzoesäureester nach Anspruch 1 oder 2, in denen einer der beiden Reste X¹ und X² H und der andere F bedeutet oder in denen die Reste gleichzeitig F bedeuten.

4. Benzoesäureester nach Anspruch 1, 2 oder 3, in denen beide Reste X¹ und X⁴ gleichzeitig F bedeuten.

5. Benzoesäureester nach Anspruch 1, 2 oder 3, in denen der Ring 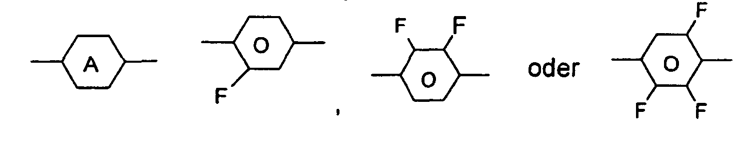 bedeutet

6. Benzoesäureester nach einem der vorhergehenden Ansprüche, in denen Y F bedeutet.

7. Benzoesäureester der Formeln I1 bis I21 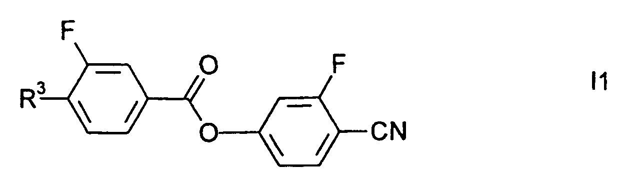 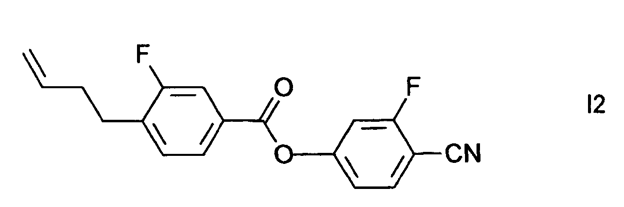 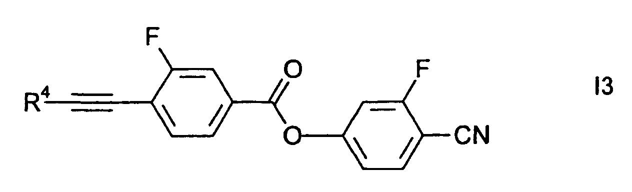 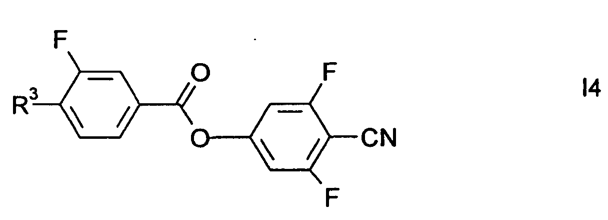 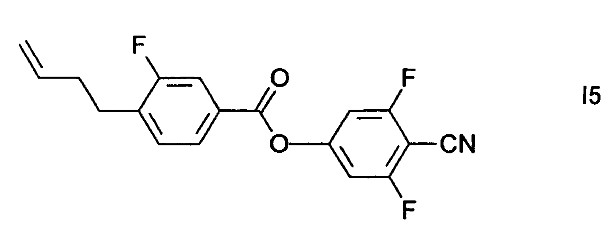 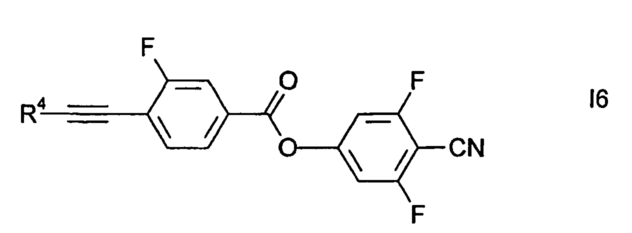 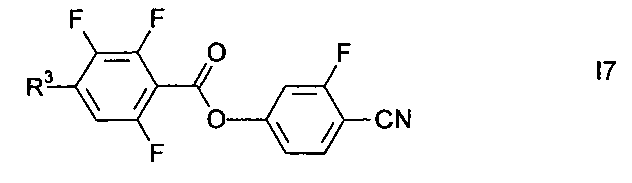 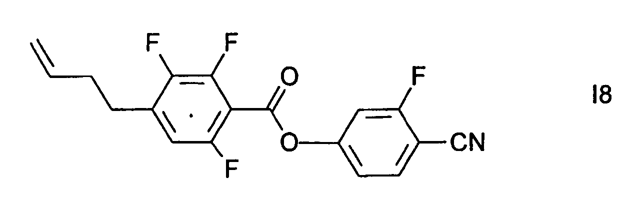 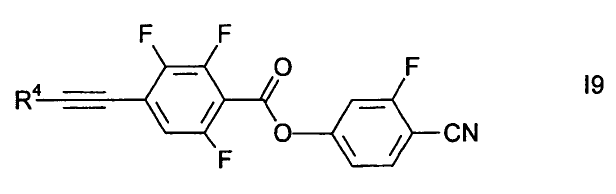 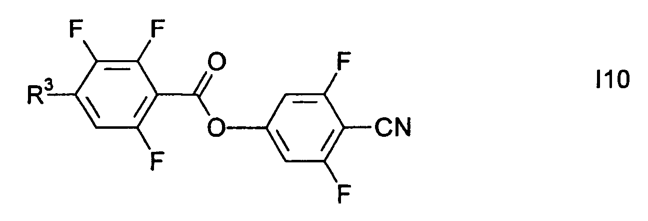 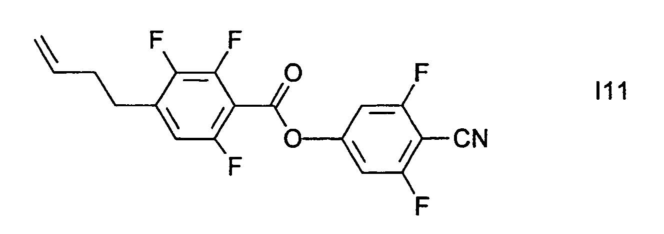 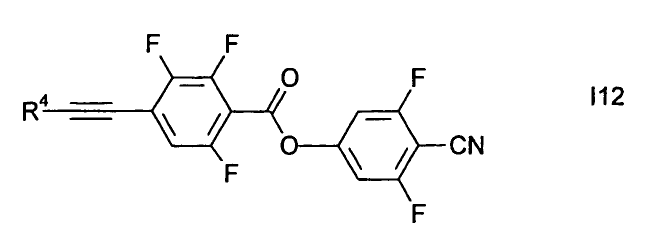 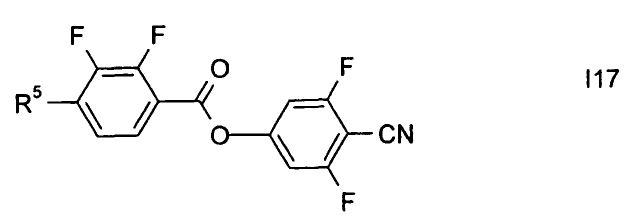 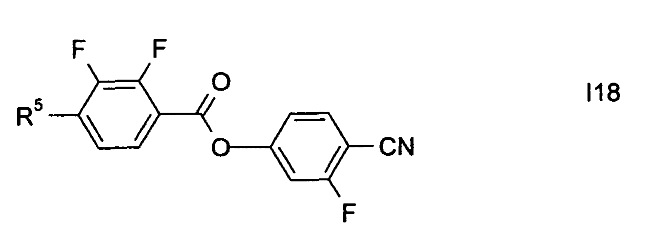 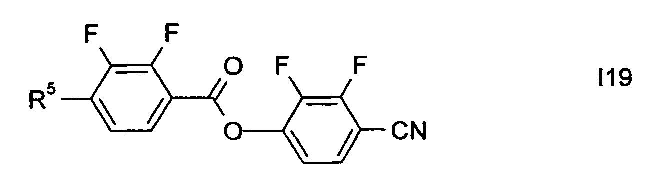 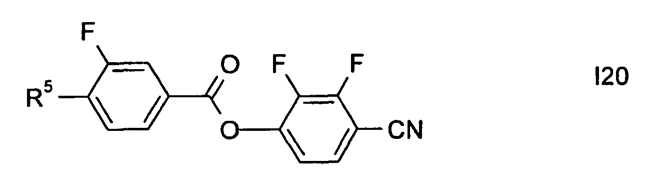 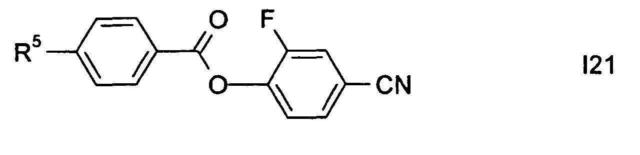 in denen X² und X⁴ unabhängig voneinander H oder F bedeuten und R³ Alkyl mit 1 bis 10 Kohlenstoffatomen, R⁴ H oder Alkyl oder Oxaalkyl mit 1 bis 7 Kohlenstoffatomen und R⁵ Alkyl oder Alkenyl mit 2 bis 7 Kohlenstoffatomen bedeutet.

8. Verwendung der Verbindungen der Formel I nach den Ansprüchen 1 bis 7 als Komponenten von flüssigkristallinen Medien.

9. Flüssigkristallines Medium mit mindestens zwei flüssigkristallinen Komponenten, **dadurch gekennzeichnet, dass** es mindestens eine Verbindung der Formel I enthält.

10. Flüssigkristall-Anzeigeelement, **dadurch gekennzeichnet, dass** es ein flüssigkristallines Medium nach Anspruch 9 enthält.

11. Elektrooptisches Anzeigeelement, **dadurch gekennzeichnet, dass** es als Dielektrikum ein flüssigkristallines Medium nach Anspruch 9 enthält.

## Revendications

1. Esters d'acide benzoïque de la formule I 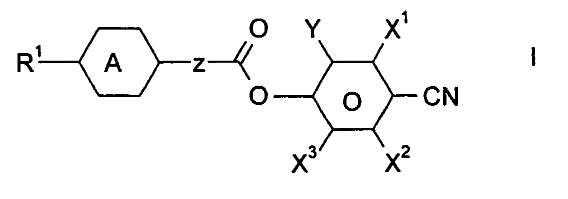 dans laquelle
R¹ est H, un radical alkyle comportant de 1 à 12 atomes de carbone, lequel est non substitué ou au moins mono- substitué par halogène ou CN et où, en plus, un ou plusieurs groupes CH₂ peuvent, chacun indépendamment les uns des autres, être remplacés par -O-, -C≡C- ou -CH=CH- de telle sorte que des hétéroatomes ne soient pas connectés directement, 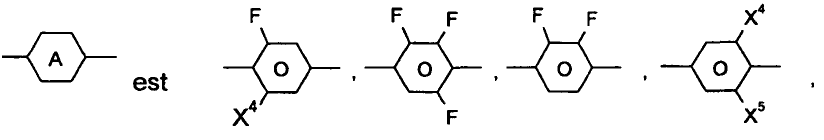 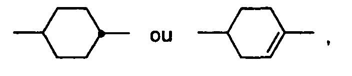
Z est -C≡C- ou une liaison simple,
Y est H ou F,
et
X¹, X², X³, X⁴ et X⁵, indépendamment les uns des autres, sont H ou F,
étant entendu que, dans les composés de la formule I
dans laquelle 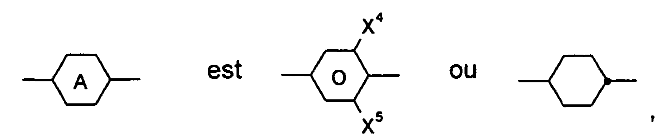
Y est F
et
a)
R¹ est un radical alkyle, oxaalkyle ou alkoxy comportant de 1 à 10 atomes de carbone où un ou plusieurs groupes CH₂ ont été remplacés par -C≡C-, et/ou
b)
Z est -C≡C-.

2. Esters d'acide benzoïque selon la revendication 1, **caractérisés en ce que** R¹ est un radical alkyle, oxaalkyle ou alkoxy comportant de 1 à 10 atomes de carbone où un ou plusieurs groupes CH₂ ont été remplacés par -C≡C-.

3. Esters d'acide benzoïque selon la revendication 1 ou 2, dans lesquels l'un des deux radicaux X¹ et X² est H et l'autre est F ou dans lesquels les radicaux sont simultanément F.

4. Esters d'acide benzoïque selon la revendication 1, 2 ou 3, dans lesquels les deux radicaux X¹ et X⁴ sont simultanément F.

5. Esters d'acide benzoïque selon la revendication 1, 2 ou 3, dans lesquels l'anneau

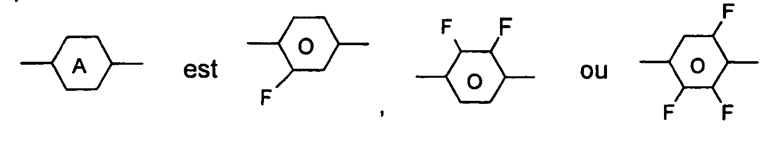

6. Esters d'acide benzoïque selon l'une quelconque des revendications précédentes, dans lesquels Y est F.

7. Esters d'acide benzoïque des formules I1 à I21 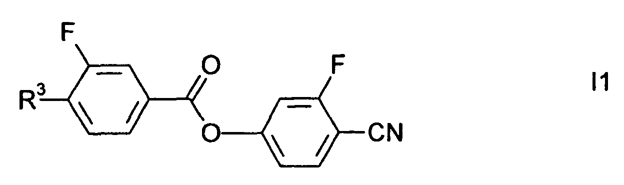 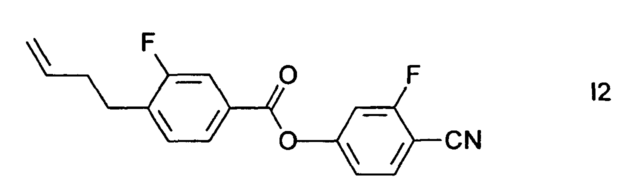 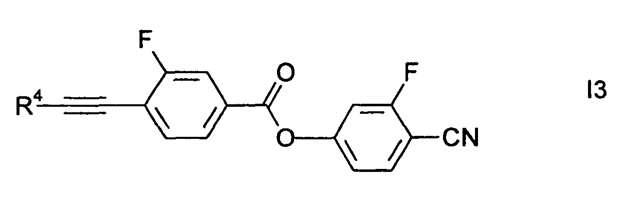 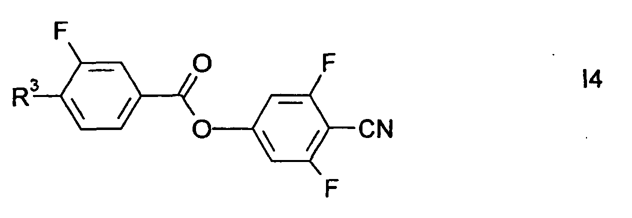 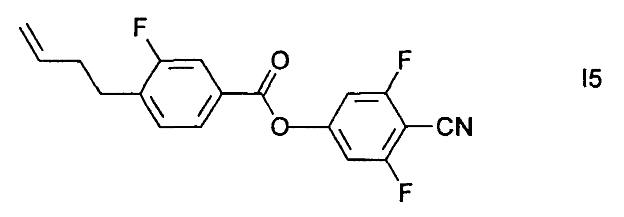 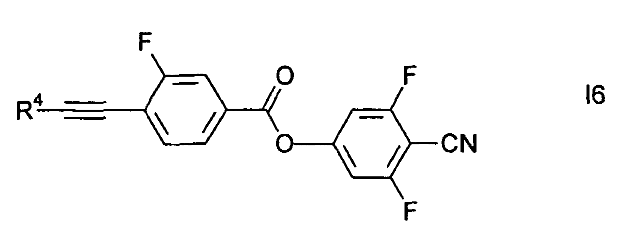 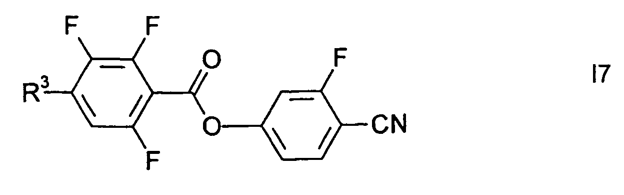 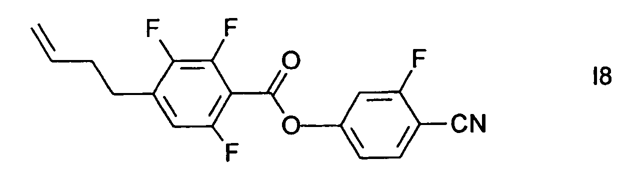 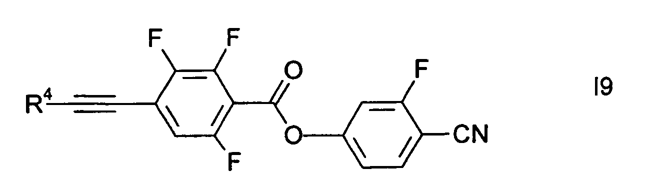 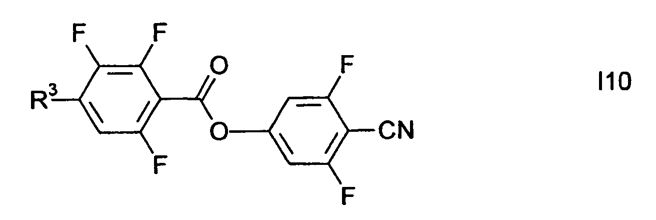 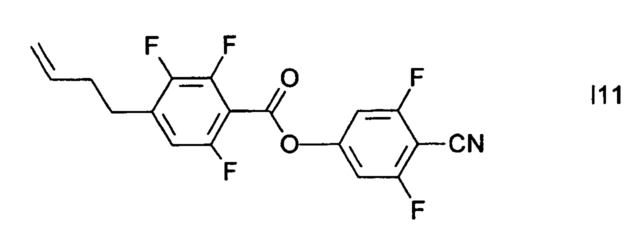 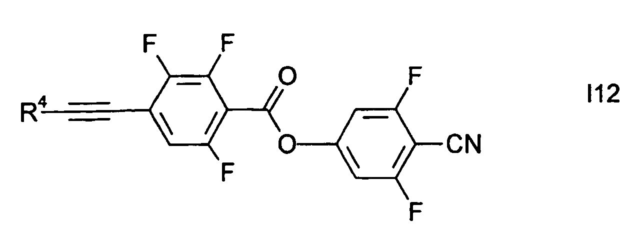 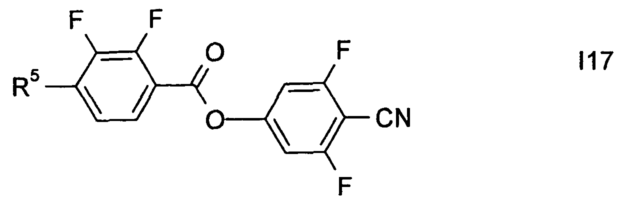 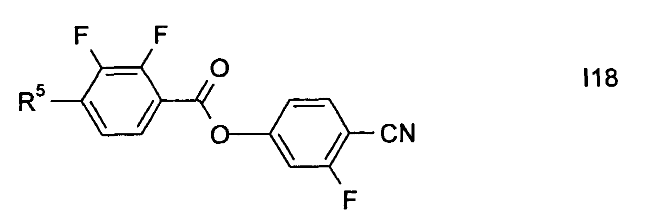 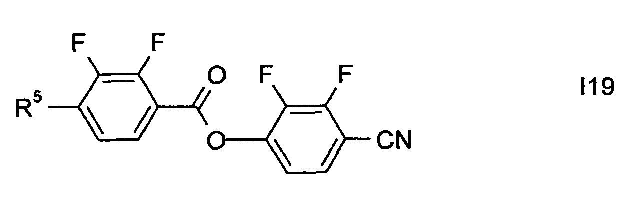 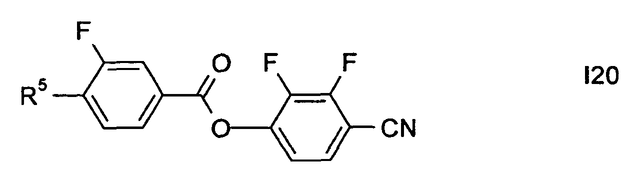 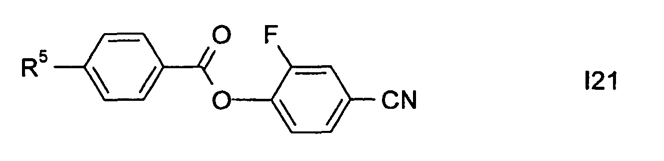 dans lesquelles X² et X⁴, indépendamment l'un de l'autre, sont H ou F, R³ est alkyle comportant de 1 à 10 atomes de carbone, R⁴ est H ou alkyle ou oxaalkyle comportant de 1 à 7 atomes de carbone, et R⁵ est alkyle ou alkényle comportant de 2 à 7 atomes de carbone.

8. Utilisation de composés de la formule I selon les revendications 1 à 7 en tant que composants de milieux de cristaux liquides.

9. Milieu de cristaux liquides comportant au moins deux composants de cristaux liquides, **caractérisé en ce qu'**il comprend au moins un composé de la formule I.

10. Elément d'affichage à cristaux liquides, **caractérisé en ce qu'**il contient un milieu de cristaux liquides selon la revendication 9.

11. Elément d'affichage à cristaux liquides, **caractérisé en ce qu'**il contient, en tant que diélectrique, un milieu de cristaux liquides selon la revendication 9.
